# EUROPEAN PATENT SPECIFICATION

(11) **EP 3 394 326 B1**
(45) Date of publication and mention of the grant of the patent: **14.09.2022**
(21) Application number: 16877036.0
(22) Date of filing: 21.12.2016
(51) Int. Cl.: C40B 30/04, C12N 5/078, C12Q 1/02, C12Q 1/68, C40B 20/00, C40B 40/02, C40B 50/06, G01N 33/543, G01N 33/566, G01N 33/574, G01N 33/68

(54) **HIGHLY PARALLEL ASSAYS FOR SIMULTANEOUS IDENTIFICATION OF ANTIBODY SEQUENCES AND BINDING PARTNERS**
HOCHPARALLELE ASSAYS ZUR SIMULTANEN IDENTIFIZIERUNG VON ANTIKÖRPERSEQUENZEN UND BINDUNGSPARTNERN
DOSAGES FORTEMENT PARALLÈLES POUR L'IDENTIFICATION SIMULTANÉE DE SÉQUENCES D'ANTICORPS ET DE PARTENAIRES DE LIAISON

(30) Priority: 21.12.2015 US 201562270094 P
(43) Date of publication of application: 31.10.2018
(73) Proprietor: The University of British Columbia, Vancouver, BC V6T 1Z3 (CA)
(72) Inventor: FALCONER, Ester, Vancouver, BC V6K 2E7 (CA); HANSEN, Carl Lars Genghis, Vancouver British Columbia V6T 1Z4 (CA); LECAULT, Veronique, Vancouver British Columbia V6Z 3C2 (CA); LISAINGO, Kathleen, Port Moody British Columbia V3H 0E4 (CA); HEYRIES, Kevin Albert, V7J 1W1 North Vancouver (CA)
(74) Representative: Novitas Patent AB
(86) International application number: PCT/CA2016/000323
(87) International publication number: WO 2017/106958

(56) References cited:
- WO-A1-2014/153651
- WO-A1-2014/153651
- US-A1- 2010 210 473
- Anupam Singhal ET AL: "A MICROFLUIDIC PLATFORM FOR SCREENING AND SELECTION OF MONOCLONAL ANTIBODIES FROM SINGLE CELLS", , 2 June 2011 (2011-06-02), XP055504387, Retrieved from the Internet: URL:http://www.rsc.org/images/LOC/2011/PDF s/Papers/109_1071.pdf [retrieved on 2018-09-04]
- V?RONIQUE LECAULT ET AL: "Microfluidic single cell analysis: from promise to practice", CURRENT OPINION IN CHEMICAL BIOLOGY, vol. 16, no. 3-4, 1 August 2012 (2012-08-01), pages 381-390, XP055287401, GB ISSN: 1367-5931, DOI: 10.1016/j.cbpa.2012.03.022
- CROWE, J.E.: 'Technical sheet for the EL4-B5 cell line sold by Kerafast (catalog number: EVU301', [Online] 24 December 2009, XP055497750 Retrieved from the Internet: <URL:HTTPS://WWW.KERAFAST.COM/PRODUCT/2291/ CD40L-EXPRESSING-FEEDER-CELL-LINE-EL-4-B5> [retrieved on 2017-02-23]
- HOOGENBOOM, H.R.: 'Selecting and screening recombinant antibody libraries''.' NATURE BIOTECHNOLOGY vol. 23, no. 9, September 2005, pages 1105 - 1116, XP002348401

## Description

### BACKGROUND OF THE INVENTION

The adaptive immune response produces a diversity of different antibodies (on the order of 10¹¹ unique sequences in a single individual), each of which is capable of binding to a different target epitope. The isolation of antibodies that bind to a specific target, let alone a specific epitope on a known target, is of high interest for the development of new products in therapeutics, diagnostics, and more generally, for research purposes. Methods for identifying such antibodies have been previously described and include hybridoma approaches, display library approaches, B-cell immortalization, B-cell cloning, and single cell screening methods. For each of these methods, it is required that the target of interest, whether it is a simple target such as a protein or a complex target such as a specific cell type, is identified and has been produced in order to perform the appropriate selections.

A much more challenging problem in antibody discovery is presented when it is known, or suspected, that an antibody response is present and is biologically relevant, but the identity of the target (and consequently, target epitope) is not known, and could be a single target selected from hundreds to tens of thousands of different targets. For example, the targets of autoantibodies are not all known; and in many instances, only a small number have been identified. In diseases and conditions in which autoantibodies are generated (e.g., autoimmune disease, cancer), the adaptive immune system escapes "tolerance", the internal checks and balances that would normally prevent the generation of antibodies that bind to self-antigens, and results in chronic inflammation, fibrosis, progressive tissue damage or a combination of the foregoing. Examples of such disorders include multiple sclerosis, myasthenia gravis, diabetes, lupus, rheumatoid arthritis, traumatic brain injury, among others. In some of these diseases, autoantigens that are associated with pathology, or which are prognostic of disease occurrence and/or disease progression have also been discovered. Further, even in the case of two patients that make autoantibodies against the same target, the sequences of these antibodies will be different. In addition, an analogous problem to autoimmune conditions occurs in cases where patients have received tissues or blood from a donor, mount an immune response against this imperfectly matched tissue, but the exact identity of the antigen responsible for the immune response is not known. As such, it is of great interest to identify autoantibodies and their respective antigens.

The present invention addresses this need, by providing methods for identifying such autoantigens and autoantibodies.

WO 2014/153651 discloses methods for determining whether a cell population comprises an antibody secreting cell (ASC) that exhibits an extracellular effect attributable to a secreted antibody of the ASC. The extracellular effect can be an effect on a readout particle population, which in one instance, can be a cell population engineered to express a cDNA library of cell surface proteins.

### SUMMARY OF THE INVENTION

In one aspect of the invention, a method is provided for determining the sequence of an antibody and its respective binding partner, e.g., antigen such as an autoantigen. The method entails optionally subjecting a sample to conditions suitable for expansion of one or more of the lymphocytes to form an expanded sample comprising antibody secreting cells (ASCs) and distributing the sample into containers to provide a plurality of subsamples. The containers are chambers of a microfluidic device. Optionally, one or more subsamples are subjected to conditions suitable for expansion of one or more ASCs or ASC precursor cells in the one or more subsamples to form one or more expanded subsamples comprising ASCs. Antibodies from one or more of the subsamples (expanded or non-expanded) are captured on a solid substrate in the respective container(s) to provided captured antibodies. The captured antibodies in one or more of the containers are exposed to an antigen display library comprising a plurality of display particles for a time sufficient to allow for interaction of one or more of the captured antibodies with the one of more of the display particles, wherein each display particle of the plurality of display particles comprises a (poly)peptide-nucleic acid complex, wherein the (poly)peptide is an antigen and the nucleic acid is used to determine the identity of the antigen.

Display particles are captured that interact with one or more of the captured antibodies. Unbound display particles are removed via a wash step.

ASCs, antibodies, display particles or a combination thereof are removed from the container(s) that contain captured display particles, and subjected to sequencing reactions. The results of the sequencing reactions determine the identity of the antibody and/or its respective antigen binding partner. In one embodiment, prior to sequencing of container contents, a determination is first made as to which containers include captured display particles. For example, in one embodiment, the contents of each of the containers are sequenced followed by a determination of the containers that contain both antibody and antigen, as well as the sequences of the antibody and antigen. In another embodiment, containers are visualized via a detection reagent to determine whether it includes bound display particles. The detection reagent can be an additional reagent, e.g., a fluorescently labeled secondary antibody that binds to a display particle. Alternatively, the display particle serves as the detection reagent. For example, the display particle in one embodiment is a fluorescently labeled display particle.

In another aspect, a method is provided for determining the sequence of an antibody and its respective binding partner, e.g., antigen such as an autoantigen. The method entails optionally subjecting a sample to conditions suitable for expansion to form an expanded sample comprising antibody secreting cells (ASCs) and distributing the sample into containers to provide a plurality of subsamples. The containers are chambers of a microfluidic device. Optionally, one or more subsamples are subjected to conditions suitable for expansion of one or more ASCs or ASC precursor cells in the one or more subsamples to form one or more expanded subsamples comprising ASCs. A functional assay is performed on one or more of the subsamples, wherein the functional assay measures a property of an antibody in the one or more subsamples. Based on the results of the functional assay, one or more functional subsamples that include one or more functional antibodies are identified. Antibodies secreted by ASCs in the one or more functional subsamples are captured on a solid substrate to provide captured antibodies. Antibody capture can be accomplished in the same container as the functional assay, or a different container. For example, a functional subsample can be transferred to a different container to perform antibody capture or alternatively transferred into a plurality of different containers to create multiple subpopulations of the transferred ASCs prior to performing the antibody capture. The captured antibodies in the containers comprising the functional subsamples are exposed to an antigen display library comprising a plurality of display particles for a time sufficient to allow for interaction of one or more of the captured antibodies with the one of more of the display particles, wherein each display particle of the plurality of display particles comprises a (poly)peptide-nucleic acid complex, wherein the (poly)peptide is an antigen and the nucleic acid is used to determine the identity of the antigen.

Display particles are captured that interact with one or more of the captured antibodies. Unbound display particles are removed via a wash step.

ASCs, antibodies, display particles or a combination thereof are removed from container(s) that contain captured display particles, and subjected to sequencing reactions. The results of the sequencing reactions determine the identity of the antibody and/or its respective antigen binding partner. In one embodiment, prior to sequencing of container contents, a determination is first made as to which containers include captured display particles. In one embodiment, the contents of each of the containers are sequenced followed by a determination of the containers that contain both antibody and antigen, as well as the sequences of the antibody and antigen. In another embodiment, containers are visualized via a detection reagent to determine whether it includes a bound display particle, prior to sequencing. The detection reagent can be an additional reagent, e.g., a fluorescently labeled secondary antibody that binds to a display particle. Alternatively, the display particle serves as the detection reagent. For example, the display particle in one embodiment is a fluorescently labeled display particle. ASCs, antibodies, display particles or a combination thereof are removed from container(s) that contain captured display particles, and subjected to sequencing reactions. The results of the sequencing reactions determine the identity of the antibody and/or its respective antigen binding partner.

In one embodiment, the sample subjected to the methods provided herein is from a patient with an autoimmune disorder. In a further embodiment, the autoimmune disorder is one of the disorders set forth in Table 1. In a further embodiment, the sample subjected to the methods provided herein is from a patient exhibiting an immune response to a transplanted organ or tissue. In one embodiment, the sample subjected to the methods provided herein is from a patient that has been exposed to an infectious agent. In another embodiment, the sample subjected to the methods provided herein is from a healthy patient. In another embodiment, the sample is derived from an animal that has been exposed to one or more of the antigens represented in the display library, or that is suspected of having mounted an immune response against one or more antigens represented in the display library.

In one embodiment, the solid substrate used in the methods provided herein is a functionalized bead or a functionalized container surface. In a further embodiment, the functionalized container surface is the surface of a microfluidic chamber. The functionalized surface in one embodiment is a protein A or a protein G functionalized surface. In another embodiment, the functionalized surface is a surface functionalized with a polyclonal antibody against human immunoglobulin or against immunoglobulin of an animal being analyzed. In another embodiment, the functionalized surface is a surface functionalized with a monoclonal antibody against human immunoglobulin. In another embodiment, the functionalized surface is a surface functionalized with a moiety that binds to human immunoglobulins of one or more isotype.

### BRIEF DESCRIPTION OF THE FIGURES

Figure 1 is a flow chart showing one aspect of the present invention (1000), *i.e.,* a method for determining the sequence of an antibody and/or its respective antigen *a priori.*
Figure 2 is a flow chart showing a second aspect of the present invention (2000), *i.e.,* a method for determining the sequence of an antibody and/or its respective antigen *a priori.*

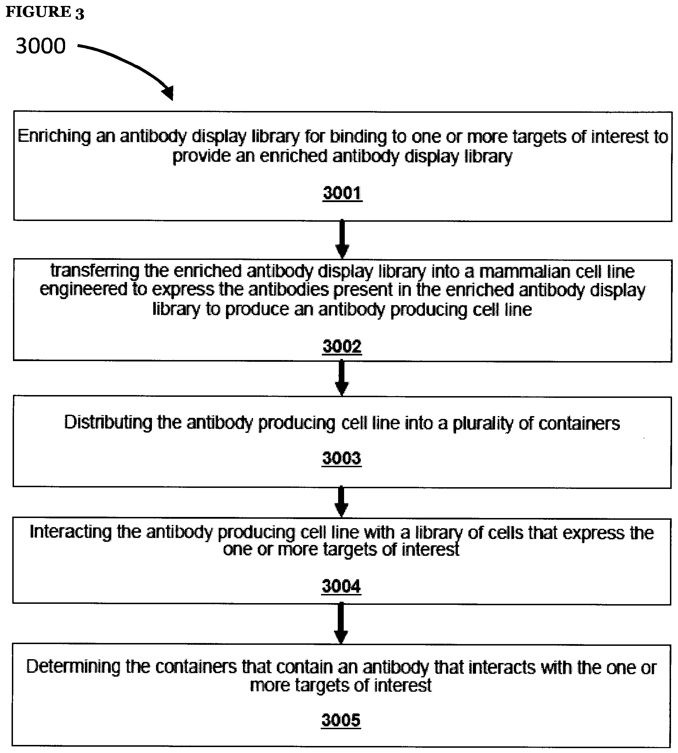

Figure 3 is a flow chart of an embodiment which is not part of the present invention (3000).
Figure 4 is an optical micrograph of a microfluidic valve fabricated by multilayer soft lithography.

### DETAILED DESCRIPTION OF THE INVENTION

As used herein, "an antibody" is a polypeptide belonging to the immunoglobulin superfamily. In particular, "an antibody" includes an immunoglobulin molecule or an immunologically active fragment of an immunoglobulin molecule (i.e., a molecule(s) that contains an antigen binding site), an immunoglobulin heavy chain (alpha (α), mu (µ), delta (δ) or epsilon (ε)) or a variable domain thereof (V_{H} domain), an immunoglobulin light chain (kappa (κ) or lambda (λ)) or a variable domain thereof (V_{L} domain), or a polynucleotide encoding an immunoglobulin molecule or an immunologically active fragment of the immunoglobulin molecule. The antibody includes a single chain antibody (e.g., an immunoglobulin light chain or an immunoglobulin heavy chain), a single-domain antibody, an antibody variable fragment (Fᵥ), a single-chain variable fragment (scFv), an scFv-zipper, an scFv-Fc, a disulfide-linked Fv (sdFv), a Fab fragment (e.g., C_{L}V_{L} or C_{H}V_{H}), a F(ab') fragment, an epitope-binding fragment of any of the above (i.e., one or more antigen binding fragments) and a polynucleotide (DNA or RNA) encoding any of the above (including a polynucleotide library encoding a plurality of antibodies). Immunoglobulin molecules can be of any type (e.g., IgG, IgE, IgM, IgD, IgA and IgY), class (e.g., IgGl, IgG2, IgG3, IgG4, IgAl and IgA2) or subclasss. The antibody may be synthetic or from any animal origin including birds and mammals (e.g., human, murine, donkey, sheep, rabbit, goat, guinea pig, camel, llama, horse, or chicken), and/or humanized versions thereof. The antibody includes a polyclonal antibody, a monoclonal antibody, a human antibody, a humanized antibody, a nanobody, a chimeric antibody, a single chain antibody, a heavy chain antibody (i.e., an antibody consisting of two heavy chains), an anti-idiotypic (anti-Id) antibody, a minibody (mini-antibody), a light chain antibody (i.e., an antibody consisting of two light chains) and a diabody, a fragment of any of the aforementioned or a polynucleotide encoding any of the aforementioned. "Antibody" can be surface bound or non-surface bound, and if surface bound, includes the surface to which it is bound (e.g., a solid support or a cell). The antibody also includes any of the aforementioned immunoglobulin molecules, or fragments thereof, complexed or bound to one or other molecules (e.g., one or antigens). A cell that secretes antibodies is referred to herein as an "antibody secreting cell" (ASC).

Specifically, an "antibody secreting cell" or "ASC," as used herein, refers to any cell type that produces and secretes an antibody. Plasma cells (also referred to as "plasma B-cells," "plasmocytes" and "effector B-cells") are terminally differentiated, and are one type of ASC. ASCs include, for example, activated memory B-cells, plasmablasts, cells generated through the expansion of memory B-cells, cell lines that express recombinant monoclonal antibodies and hybridoma cell lines. Typically, each ASC has a diameter of from about 7 µm to about 15 µm, depending on source, a diameter approximately 1/10^{th} the width of a human hair, and generates only a minute amount of antibody. When analyzed in the volume of conventional formats this small amount of antibody is typically too dilute, making it difficult to analyze its properties by the majority of existing methods. For this reason, antibody discovery typically requires that each ASC be isolated, fused to an immortal cancer cell to create a hybridoma and "grown," ultimately generating many thousands of identical cells that can produce enough antibody to be measured. For example, see McCullough and Spier (1990). Monoclonal Antibodies in Biotechnology: Theoretical and Practical Aspects, Chapter 2, Cambridge University Press). This process is not only inefficient (99% of the starting immune cells are lost) but also very slow and expensive, requiring at minimum 3 months of labor before therapeutic function can be tested.

In various aspects of the invention, the methods described herein provide a means to simultaneously screen a panel of antibodies in a plurality of different containers (e.g., from about 100 to about 10,000,0000 different containers, each containing from about 0 to about 10,000 unique ASCs), each secreted by a different cell or subset of cells, against a library of possible target epitopes (e.g., a library of from about 10 to about 10¹² unique target antigen epitopes). The methods provided herein can be used in various embodiments, (i) to isolate high-value antibodies from one or more libraries, (ii) to understand antibody-antigen interactions, (iii) to optimize antibody sequence, (iv) to evaluate epitope binding properties of antibodies, (v) to screen antibodies expressed and secreted by engineered mammalian cells, (vi) to isolate antibodies from immunized animals or animals exposed to one or more members of the display library, or (vii) a combination thereof. For example, in one embodiment, the methods provided herein are used in the context of infectious diseases to find antibodies against a virus or bacterium without knowing *a priori* the target antigen(s). In another embodiment, the methods provided herein are carried out in the context of autoimmune disease or cancer, to find novel antibody(s) and/or novel antigen(s) associated with the respective autoimmune disease or cancer. In another embodiment, the methods described herein are used to isolate antibodies associated with a functional response that have been produced by an animal immunized with one or more antigens. As there is evidence to suggest that different types of cancer trigger unique autoantibody signatures that reflect the nature of the malignant process in the affected organ, a need exists for methods to identify autoantibody signatures (Casiano et al. (2006). Molecular & Cellular Proteomics 5.10, pp. 1745-1759).

The value of finding new antigens, e.g., autoantigens is multifold. For example, the methods provided herein have the potential to provide the following benefits: (i) improved understanding of disease progression, e.g., autoimmune disease and cancer (tumor response, metastasis, etc.); (ii) improved methods and tests for diagnosis of autoimmune disease; (iii) identification of therapeutic targets for the treatment of for example, cancer and/or autoimmune disease (iv) informing the design of therapeutics that block the association of autoantibodies by mimicking the epitopes involved; and (v) stratification of patients based on autoimmune reactivity, (vi) the design and evaluation of responses to vaccines in humans and animals. Similarly, newly discovered antibodies that react against self-antigens may be used as reagents in diagnostics, or repurposed as therapeutics to inhibit the destructive effects of autoantibodies.

In one aspect, provided herein is a high-throughput and sensitive method for (i) isolating the sequences of antibodies that react to antigens, e.g., self-antigens, without knowledge of the identity of the antigen *a priori,* and/or (ii) discovering the identity of antigen(s) that mediate a disease, or whose presence is correlated with a disease state, e.g., an autoantigen(s) that mediates an autoimmune disorder(s), or whose presence is correlated with the presence of an autoimmune disorder(s), an antigen(s) that mediates a tumor response.

The methods described herein can be applied to the identification of antibodies that react with particular antigens, either native or mutated. The antigen can be, in various embodiments, an antigen implicated in a tumor or a metastatic response, autoimmune disease, in a response to a bacterium or virus (or any foreign substance), in response to immunization or vaccination, or that are elicited in the context of organ or tissue transplantation. Immune cells (ASCs or ASC precursor cells) can be isolated from cancer patients that have mounted an effective immune response to a cancer, either spontaneously or in conjunction with immune-therapy treatments such as cancer vaccines, oncolytic viruses, immune-modulators and/or checkpoint inhibitors, and subjected to one of the methods described herein.

The methods provided herein are based in part on the combination of two approaches. The first is the use of a display library that links phenotype (e.g., a peptide or protein antigen) to its genotype (coding sequence) such as is accomplished in formats such as phage display, other cell display methods (bacterial, yeast, etc.), ribosome display, mRNA display, cDNA display, bead display particles or other particles to determine antibody binding to antigen. Here, an isolated protein in a microscale container, e.g., a secreted antibody (or antigen binding fragment thereof) is used to enrich for display particles that present an epitope displayed on a subset of the library particles, followed by sequencing of the DNA or RNA information in the particles to determine the identity of the enriched antigen(s). A related approach has been used in a "library versus library" strategy where two sets (libraries) of display particles presenting different families of molecules, for example, yeast cells expressing antibody fragments on their surface and phage displaying peptide libraries, are mixed together and incubated to promote an association of the subset(s) of particles from each library that interact. Following incubation, sequencing is used to determine identification of the antibodies and antigens that are associated. The present invention differs from the library versus library approach in that it is not limited to the use of fabricated antibody libraries. Rather, it harnesses the capture of secreted antibodies from a clinically or scientifically relevant environment and the ability to isolate the cell which secretes the antibody. Moreover, antibodies used in the present invention are not physically linked to their respective genetic material, as is done with the library versus library approach (i.e., where antibody display libraries are harnessed).

A second technology harnessed by the methods provided herein is single cell screening (or in some cases a small population of cells, e.g., from about 2 cells to about 100 cells), based on the analysis of secreted antibodies from a single ASC, using local capture of these antibodies on solid supports in microscale volumes and containers. In this regards, single cell sensitivity in measuring the presence and/or properties of secreted antibodies from an individual ASC is obtainable even when multiple unique ASCs are present in the same microscale volume (in the same container). Stated another way, because each ASC can produce high concentrations of secreted antibody within minutes, the ASC can be present in a population of cells within a container, and its respective secreted antibody can still be captured via an antigen display particle, and such an interaction can be deciphered.

In one aspect, the present invention provides a method for screening a diverse set of antibodies, or other antigen-binding proteins, against a library of potential antigens. In this regard, the present invention can be used to identify autoantigens implicated in various disease states by screening ASCs (or other ASC precursor lymphocytes that are subsequently differentiated into ASCs) harvested from a patient that has an autoimmune disease against a library of antigens. In one embodiment, the output of the method provided herein is the determination of the nucleic acid or protein sequence of one or more antibodies (or antigen binding fragments thereof) that recognize a member of the library of antigens, and/or the nucleic acid or protein sequence of the antigen that is recognized. As such, the method provided herein can be used to screen lymphocytes (e.g., ASC precursor cells or ASCs such as plasma cells, plasmablasts, etc.) obtained from various tissues of patients that have an autoimmune disorder or cancer, allowing for the discovery, *a priori,* of antibodies that bind to self-antigens (autoantigens) as well as the autoantigens that bind to these antibodies.

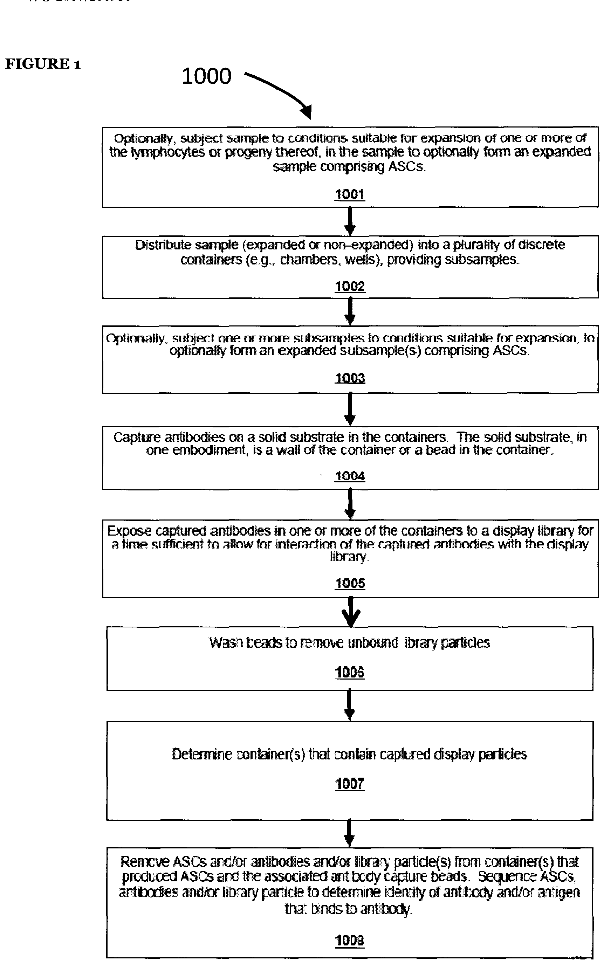

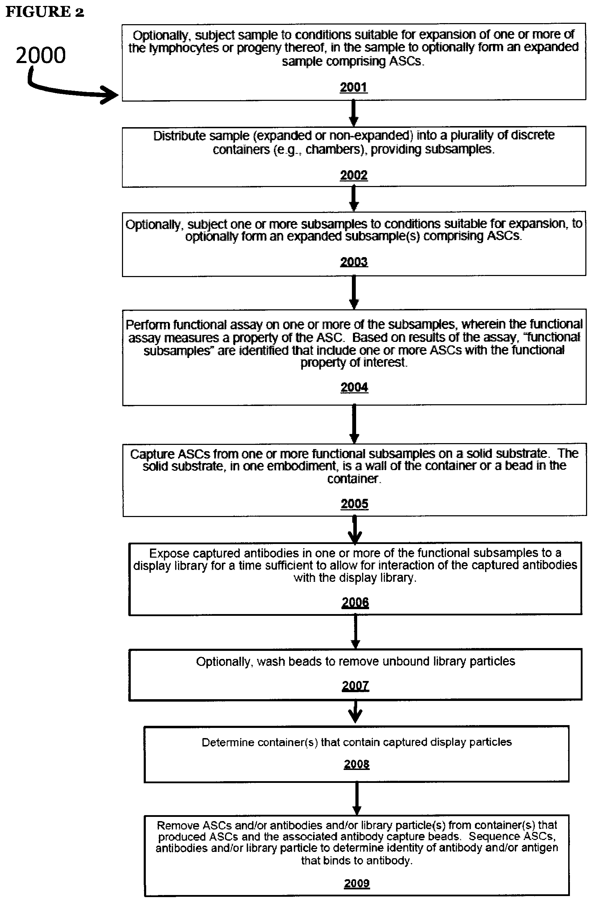

Aspects of the invention, methods **1000** and **2000** are provided at Figures 1 and 2, respectively, and discussed in further detail below. In one aspect of the invention, a method 1000 is provided for determining the sequence of an antibody and its respective binding partner, e.g., antigen such as an autoantigen. The method entails optionally subjecting a sample to conditions suitable for expansion of one or more ASCs or ASC precursor cells in the sample to form an expanded sample comprising antibody secreting cells (ASCs) **1001.** The sample or expanded sample is distributed into containers to provide a plurality of subsamples **1002.** Optionally, one or more subsamples, each comprising one or more ASCs or ASC precursor cells, are subjected to conditions suitable for expansion to form one or more expanded subsamples comprising ASCs **1003.** Antibodies from one or more of the subsamples (expanded or non-expanded) are captured on a solid substrate in the respective container(s) to provided captured antibodies **1004.** The captured antibodies in one or more of the containers are exposed to an antigen display library comprising a plurality of antigen display particles for a time sufficient to allow for interaction of one or more of the captured antibodies with the one of more of the display particles **1005.** Display particles are captured that interact with one or more of the captured antibodies. Unbound display particles are removed via a wash step **1006.** The nucleic acid sequence(s) of antibodies or antigen fragment binding portion(s) thereof, and/or antigens in containers that include captured antigen display particles is then determined **1007.** Each of the steps and components of method **1000** are discussed in further detail below.

In another aspect, a method **2000** is provided for determining the sequence of an antibody and its respective binding partner, e.g., antigen such as an autoantigen. The method entails optionally subjecting a sample to conditions suitable for expansion of one or more ASCs or ASC precursor cells in the sample to form an expanded sample comprising antibody secreting cells (ASCs) **2001.** The sample or expanded sample is distributed into containers to provide a plurality of subsamples **2002.** Optionally, one or more subsamples, each comprising one or more ASCs or ASC precursor cells, are subjected to conditions suitable for expansion to form one or more expanded subsamples comprising ASCs **2003.** A functional assay that measures a property of an antibody or ASC is performed on one or more of the subsamples **2004.** The functional assay **2004** is performed to identify a subsample(s) that exhibits the functional property of interest (i.e., to identify "functional subsamples"). Antibodies secreted by ASCs in the one or more functional subsamples are captured on a solid substrate to provide captured antibodies **2005.** Antibody capture can be accomplished in the same container as the functional assay, or a different container. For example, a functional subsample can be transferred to a different container to perform antibody capture or alternatively transferred into a plurality of different containers to create multiple subpopulations of the transferred ASCs prior to performing the antibody capture. The captured antibodies in the containers comprising the functional subsamples are exposed to an antigen display library comprising a plurality of antigen display particles for a time sufficient to allow for interaction of one or more of the captured antibodies with the one of more of the antigen display particles **2006.** Display particles are captured that interact with one or more of the captured antibodies. Unbound display particles are removed via a wash step **2007.** The nucleic acid sequence(s) of antibodies or antigen fragment binding portion(s) thereof, and/or antigens in containers that include captured display particles is then determined **2008.** Each of the steps and components of method **2000** are discussed in further detail below.

The sample used in the methods presented herein **1000** and **2000,** contains one or more ASCs or ASC precursor cells. The sample can be subjected to conditions suitable for expansion to produce ASCs from precursor lymphocytes at various steps of the methods provided herein, as described in further detail below.

The one or more ASCs or ASC precursor cells in the sample, in one embodiment, include one or more ASCs, such as one or more plasma cells (also referred to as "plasma B-cells," "plasmocytes" and "effector B-cells"). Plasma cells are terminally differentiated, and are one type of ASC. ASCs include, for example, activated memory B-cells, plasmablasts, cells generated through the expansion of memory B-cells, cell lines that express recombinant monoclonal antibodies and hybridoma cell lines.

In one embodiment of the method **1000** or **2000,** the source of the sample is a mammal. For example, the sample can be obtained from a human, e.g., a human patient that has a disease or infection. In one embodiment, the sample source is a non-human primate. In a further embodiment, the sample is a chimpanzee, gorilla, orangutan or baboon sample. Other sources of samples include, but are not limited to a human, rat, mouse, rabbit, alpaca, camel, dog, goat, bovine, gerbil, guinea pig, hamster, llama, pig or sheep. In one embodiment, the biological source of the sample is a non-mammalian vertebrate such as an avian or reptilian species. The sample used herein can be obtained from a variety of tissues including, but not limited to, bone marrow, spleen, blood, lymph nodes. The sample may also be obtained from B-cell infiltrates or structures isolated from tumors or tissues associated (e.g., correlated) with the particular disease state (e.g., brain and/or pancreas).

The sample used herein, once obtained from a source, can be further purified or modified prior to use in the methods provided herein. For example, ASCs or ASC precursor cells, B-cells or plasma cells may be enriched using methods that are known to those of ordinary skill in the art including bead-based affinity enrichment, flow cytometry and sorting, or density centrifugation, and further subjected to the methods described herein. For example, the one or more ASCs or ASC precursor cells from the sample in one embodiment, are purified from a sample obtained from an *in vivo* or *in vitro* source. In another embodiment, the obtained sample is expanded via monoclonal or polyclonal activation, as described below and/or by methods known to those of ordinary skill in the art.

In yet another embodiment, the sample is a recombinant cell line that includes cells engineered to express antibodies, or yeast engineered to express antibodies, or bacteria engineered to express antibodies.

In one embodiment, ASCs are produced by activating B-cells obtained from a sample or by amplifying and transferring the DNA content within B-cells to another cell line that can recombinantly express the antibodies. The produced ASCs are then further subjected to the method steps (e.g., method **1000** or method **2000,** see Figures 1 and 2) described herein.

In one embodiment, ASCs are obtained directly from a blood, primary lymphoid tissue or peripheral lymphoid tissue sample. The sample may be selected in order to enrich for antibodies associated with a particular condition that the user of the method desires to analyze, for example, an autoimmune disease or cancer. Primary lymphoid tissue samples amenable for use with the methods provided herein include bone marrow samples. Secondary lymphoid tissues amenable for use with the methods provided herein include but are not limited to, tonsils, spleen, lymph nodes, Peyer's patches, mucosa associated lymphoid tissue (MALT), gut associated lymphoid tissue (GALT), or a combination thereof. In another embodiment, a thyroid, cerebral spinal fluid, brain tissue, or tumor tissue sample is used with the methods provided herein. Appropriate tissue type can also be selected by one of ordinary skill in the art based on a patient's diagnosis and/or prognosis.

In one embodiment of the invention, the methods described herein are used to identify an autoantibody and/or autoantigen associated with an autoimmune disorder or cancer. In a further embodiment, the sample interrogated by a method provided herein is from an autoimmune patient, for example, a patient diagnosed one of the autoimmune disorders set forth in Table 1. The sample as provided above, can be obtained from blood, primary lymphoid tissue, peripheral or a secondary lymphoid tissue (e.g., lymph nodes, lymphoid follicles in tonsils, Peyer's patches, spleen, adenoids, skin) sample.

In another embodiment, the sample is obtained from a subject having one of the following autoimmune disorders: alopecia areata, autoimmune hemolytic anemia, autoimmune hepatitis, dermatomyositis, diabetes (type 1, some forms of type 2 - MODY), a form of juvenile idiopathic arthritis, glomerulonephritis, Graves' disease, Guillain-Barre syndrome, idiopathic thrombocytopenic purpura, myasthenia gravis, some forms of myocarditis, multiple sclerosis, pemphigus/pemphigoid, pernicious anemia, polyarteritis nodosa, polymyositis, primary biliary cirrhosis, psoriasis, rheumatoid arthritis, scleroderma/systemic sclerosis, Sjogren's syndrome, systemic lupus erythematosus (lupus), some forms of thyroiditis, some forms of uveitis, vitiligo, or granulomatosis with polyangiitis (Wegener's). In these embodiments, the sample can be further expanded (e.g., Figure 1, **1001** or **1003;** Figure **2****, 2001** or **2003**) or analyzed without an expansion step, as described below.

| **Table 1 - Autoimmune Disorders Amenable for Analvsis bv the Methods Provided Herein** | | | |
|---|---|---|---|
| Acute disseminated encephalomyelitis (ADEM) | Dermatitis herpetiformis | Linear IgA disease (LAD) | Psoriatic arthritis |
| Acute necrotizing hemorrhagic leukoencephalitis | Dermatomyositis | Lupus (SLE) | Idiopathic pulmonary fibrosis |
| Addison's disease | Devic's disease (neuromyelitis optica) | Lyme disease, chronic | Pyoderma gangrenosum |
| Agammaglobulinemia | Discoid lupus | Menier's disease | Pure red cell aplasia |
| Alopecia areata | Dressler's syndrome | Microscopic polyangiitis | Raynauds phenomenon |
| Amyloidosis | Endometriosis | Mixed connective tissue disease (MCTD) | Reactive arthritis |
| Ankylosing spondylitis | Eosinophilic esophagitis | Mooren's ulcer | Reflex sympathetic dystrophy |
| Anti-GBM/Anti-TBM nephritis | Eosinophilic fasciitis | Mucha-Habermann disease | Reiter's syndrome |
| Antiphospholipid syndrome (APS) | Erythema nodosum | Multiple sclerosis | Relapsing polychondritis |
| Autoimmune angioedema | Experimental allergic encephalomyelitis | Myasthenia gravis | Restless leg syndrom |
| Autoimmune aplastic anemia | Evans syndrome | Myositis | Retroperitoneal fibrosis |
| Autoimmune dysautonomia | Fibromyalgia | Narcolepsy | Rheumatic fever |
| Autoimmune hepatitis | Fibrosing alveolitis | Neuromyelitis optica (Devic's) | Rheumatoid arthritis |
| Autoimmune hyperlipidemia | Giant cell arteritis (temporal arteritis) | Neutropenia | Sarcoidosis |
| Autoimmune immunodeficiency | Giant cell myocarditis | Ocular cicatricial pemphigoid | Schmidt syndrome |
| Autoimmune inner ear disease (AIED) | Glomerulonephritis | Optic neuritis | Scleritis |
| Autoimmune myocarditis | Goodpasture's syndrome | Palindromic rheumatism | Scleroderma |
| Autoimmune oophoritis | Hashimoto's encephalitis | PANDAS (Pediatric autoimmune neuropsychiatric disorders associated with Streptococcus) | Sjogren's syndrome |
| Axonal & neuronal neuropathies | Hashimoto's thyroiditis | Paraneoplastic cerebellar degeneration | Sperm & testicular autoimmunity |
| Balo disease | Hemolytic anemia | Paroxysmal nocturnal hemoglobinuria (PNH) | Stiff person syndrome |
| Behcet's disease | Henoch-Schonlein purpura | Parry Romberg syndrome | Subacute bacterila endocarditis (SBE) |
| Bullous pemphigoid | Grave's disease | Parsonnage-Turner syndrome | Susac's syndrome |
| Cardiomyopathy | Guillain Barre syndrome | Pars planitis (peripheral uveitis) | Sympathetic ophthalmia |
| Castleman disease | Herpes gestationis | Pemphigus | Takayasu's arteritis |
| Celiac disease | Hypogammaglobulinem ia | Peripheral neuropathy | Temporal arteritis/Giant cell arteritis |
| Chagas disease | Idiopathic thrombocytopenic purpura (ITP) | Perivenous encephalomyelitis | Thrombocytopenic purpura (TTP) |
| Chronic fatigue syndrome | IgA neuropathy | Pernicious anemia | Tolosa-Hunt syndrome |
| Chronic inflammatory demyelinating polyneuropathy (CIDP) | IgG4-related sclerosing disease | POEMS syndrome | Transverse myelitis |
| Chronic recurrent multifocal ostomyelitis (CRMO) | Immunoregulatory lipoproteins | Polyarteritis nodosa | Type 1 diabetes |
| Churg-Strauss syndrome | Inclusion body myositis | Type I, II, & III autoimmune polyglandular syndromes | Type 1.5 diabetes (LADA) |
| Cicatricial pemphigoid/benign mucosal pemphigoid | Interstitial cystitis | Polymyalgia rheumatica | MODY diabetes (types...) |
| Crohn's disease | Juvenile arthritis | Polymyositis | Ulcerative colitis |
| Cogans syndrome | Juvenile myositis | Postmycocardial infarction syndrome | Undifferentiated connective tissue disease (UCTD) |
| Cold agglutinin disease | Kawasaki syndrome | Postpericardiotomy syndrome | Uveitis |
| Congenital heart block | Lambert -Eaton syndrome | Progesterone dermatitis | Vasculitis |
| Coxsackie myocarditis | Leukocytoclastic vasculitis | Primary biliary cirrhosis | Vesiculobullous dermatosis |
| CREST disease | Lichen planus | Primary sclerosing cholangitis | Vitiligo |
| Essential mixed cryoglobulinemia | Lichen sclerosus | Psoriasis | Wegener's granulomatosis (aka Granulomatosis with polyangiitis (GPA)) |
| Demyelinating neuropathies | | | |

Once the sample is obtained, as an optional step **1001** or **2001,** the sample comprising one or more ASCs or ASC precursor cells, e.g., a sample obtained from a subject, a sample obtained from a subject that is subjected to cell sorting to contain a substantially purified lymphocyte population, or an *in vitro* sample, is subjected to conditions suitable for expansion of the ASCs or ASC precursor cells in the sample, to obtain an expanded sample comprising antibody secreting cells (ASCs) (e.g., plasmablasts or plasma cells). Determination of whether expansion is carried out can be decided by the user of the method, for example, depending on the original sample type and the types of cells in the sample.

In some embodiments, the cells from a sample may be expanded in a cell culture step in order to increase the number of ASCs expressing any given antibody. This may be done prior to **(1001** or **2001)** and/or subsequent to partitioning **(1003** or **2003)** the sample into subsamples. The expansion step may be used to increase the generated concentration of any given antibody within a sample container in order to obtain sufficient amounts of antibody to interact with antigen library particles or to implement a functional assay. Conditions suitable for expansion include both conditions for polyclonal expansion and conditions for antigen-specific expansion, as described in further detail below. In some embodiments, the ASCs may be fused with an appropriate cell line to create hybridoma cells that can be expanded to a greater extent. Methods for the generation of hybridoma cells from a variety of species, including mice, rats, gerbils, humans, and rabbits, are known to those of ordinary skill in the art. In some embodiments an alternative immortalization method, such as Epstein-Barr virus (EBV) immortalization, may be used instead of hybridoma methods, as is known to those of ordinary skill in the art.

ASCs in one embodiment are produced by activating B-cells obtained from a tissue sample or by amplifying and transferring the DNA content within an ASC to another cell line that can recombinantly express the antibodies secreted by the ASC. In some embodiments, the cells from the tissue are expanded in order to increase the number of ASCs in a sample. An expansion step may be done prior to partitioning a sample into subsamples (where a subsample can comprise 0 or a plurality of ASCs) in discrete chambers **(1001** or **2001),** or after partitioning **(1003** or **2003).**

Activation of the sample leads to the formation of an expanded sample. In one embodiment, activation is carried out in cell culture of the one or more ASCs or ASC precursor cells in the sample (which can be subjected to one or more purification steps such as FACS). In one embodiment, in the case of a heterogeneous immune cell population, activation is employed for the entire population of lymphocytes or progeny thereof, *e.g.,* through polyclonal activation. Alternatively or additionally, activation is carried out on a subpopulation of cells, *e.g.,* with antigen specific activation and subsequent expansion. In one embodiment, where a sample includes a combination of B-cells and T-cells, only the B-cells (or a subpopulation thereof) are activated, or both the B-cells (or a subpopulation thereof) and the T-cells (or a subpopulation thereof) are activated and expanded, to form an expanded sample **1001** or **2001,** or an expanded subsample **1003** or **2003.**

Methods for performing antigen-specific expansion of B-cells and T-cells are known to those of ordinary skill in the art and the present invention is not limited by a particular expansion method. Rather, an expansion step can be carried out according to a protocol determined by the user of the method. Activation of a lymphocyte or progeny thereof causes the activated cell to divide and expand to form an expanded sample comprising an ASC (or an expanded subsample). Both polyclonal activation and antigen-specific activation are amenable for use with the present methods. The activation/expansion step can be carried out according to a protocol determined by the user of the method.

In one embodiment, a CD40L expressing feeder cell line (e.g. EL-4-B5) is used to activate and expand B-cells, and to produce ASCs. The EL-4-B5 cells express CD40-ligand and are grown with B-cells in the presence of T cell supernatants to activate the B-cells via direct cell contact to induce proliferation, differentiation, and secretion of antibody. The EL-4-B5 cell line is available commercially, for example, from Kerafast (catalog no. EVU301). In another embodiment an antibody that engages and activates CD40 may be used in the activation step, as is known to those of ordinary skill in the art.

B-cells residing primarily in peripheral lymphoid tissues in one embodiment, are activated and expanded into antibody-secreting cells (ASCs) upon antigen stimulation. In one embodiment, B-cells are activated under defined *in vitro* culture conditions resulting in polyclonal expansion and differentiation into ASCs. In the case of memory B-cells, activation and expansion in one embodiment is accomplished by treating the cells with, for example, Epstein Barr virus, CD40L, or one or more toll like receptor agonists, using protocols that are known to those of ordinary skill in the art. Protocols described in the literature that may be used to induce B-cell activation by adding supplements in the cell culture media are amenable for use with the present invention. These include different combinations of factors such as cytokines *(e.g.,* IL-21, IL-6, IL-4, IL-2, IL-10, IL-15) (Ettinger et al. (2005). The Journal of Immunology 175, pp. 7867-7879; Pinna et al. (2009). Eur. J. Immunol. 39, pp. 1260-1270 and Bernasconi et al. (2002). Science 298, pp. 2199-2202); cell surface ligands *(e.g.,* CD40L, BAFF, APRIL), toll-like receptor agonists (e.g. LPS, CpG, R848, PWM) (see, e.g., Pinna et al. (2009). Eur. J. Immunol. 39, pp. 1260-1270; Boeglin et al. (2011). PLOS One 6, p. e25542. doi:10.1371/journal.pone.0025542; Hartmann and Krieg (2000). J. Immunol. 164, pp. 944-953; Krieg et al. (1995). Nature 374, pp. 546-549; Crotty et al. (2004). J. Immunol. Methods 286, pp. 111-122; Endoh et al. (1987). Cell Immunol. 107, pp. 455-464), monoclonal antibodies against cell surface receptors (e.g. anti-CD40, anti-IgG) (Zhu et al. (2002). J. Immunol. 168, pp. 744-754; Endoh et al. (1987). Cell Immunol. 107, pp. 455-464), and feeder cell lines providing co-stimulation signals (e.g., cell lines expressing CD40L) (Seeber et al. 2014) PLOS One 9, e86184. doi:10.1371/journal.pone.0086184; Wen et al. (1987). Eur. J. Immunol. 17, pp. 887-892; Liebig et al. (2009). J. Vis. Exp. 16, pii: 1373. Doi: 10.3791/1373).

In one embodiment, one or more activated cells in the expanded sample **1001** or **2001** are purified and/or enriched for. Purification/enrichment can be carried out according to methods known to those of ordinary skill in the art. In one embodiment, purification of activated cell(s) is carried out based on identification of cellular morphology or expansion marker(s), a FACS secretion assay, purified or enriched, a Milteny kit (e.g., IFN-y kit or custom), cytokine secretion assays, cell marker assay wherein the cell marker is turned on upon activation, peptide-based purification by FACS, or a combination thereof.

The sample (e.g., purified, expanded, enriched) is distributed into a plurality of discrete containers (microfluidic chambers) (Figure 1, **1002,** Figure 2, **2002).**

In the methods provided herein, the sample (e.g., cultured sample, expanded, purified, or directly from a source) is distributed into a plurality of discrete containers **1002** or **2002.** The discrete containers (also referred to as vessels) are microfluidic chambers, for example, the microfluidic chambers described in PCT Publication No. WO 2014/153651.

In one embodiment, distributing the sample into discrete containers **1001** or **2002** results in a large number of ASCs that are maintained in spatial proximity to a substrate functionalized to capture the secreted antibodies from the ASCs, e.g., functionalized microbeads. In one embodiment, ASCs are obtained directly from a tissue sample which may be first selected in order to enrich for antibodies associated with a particular autoimmune condition. Such tissues may include, but are not limited to, tonsils, blood, bone marrow, spleen, lymph nodes, and cerebral spinal fluid. In another embodiment, a sample containing ASCs or ASC precursor cells is activated and expanded prior to distribution into containers. In some embodiments, the cells are grown with a thymoma cell line such as the murine EL4 thymoma cell line, which optionally, may have been mutagenized. For example, the thymoma cell line may have been mutagenized to obtain bromo-deoxyuiidine-resistani mutants (e.g., EL4-B5 cells, e.g., the cell line available from Kerafast, Inc., Boston, MA, catalog no. EVU301). In some embodiments, the cells are grown with macrophages (e.g., PD3188 cells or P388D1 cells) and/or T-cells. In a further embodiment, expansion occurs via the use of an EL-4-B5 feeder cell line.

In the methods described herein, a sample comprising ASCs or ASC precursors is distributed into individual containers (microfluidic chambers) **(1002** or **2002).** As used herein, a "container" is used interchangeably with a "vessel." A container is an individual chamber in a microfluidic device. In one embodiment, the sample (e.g., an expanded sample) is split into at least about 50, at least about 100, at least about 150, at least about 200, at least about 250, at least about 300, at least about 350, at least about 400, at least about 500, at least about 600, at least about 700, at least about 800, at least about 900, at least about 1000, at least about 2000, at least about 5000 discrete containers, at least 20,000 discrete containers, at least 100,000 discrete containers, at least 200,000 discrete containers, at least 500,000 discrete containers or at least 1,000,000 discrete containers (microfluidic chambers) for further processing. The number of ASCs and/or ASC precursors in each container can be the same or different. Moreover, tissues from multiple patients can be analyzed on an individual plate or device comprising multiple containers.

In one embodiment, each subsample can contain from on average, about a single cell to about 10,000 cells (ASCs or other lymphocyte cell(s)), depending on the capacity of the chamber used. It will be appreciated that microscale chambers will have less capacity than macroscopic chambers and that appropriate cell densities will be different. It should be noted that each container need not contain an ASC or ASC precursor cell. Accordingly, in some embodiments, and depending on the number of cells in the original sample and/or whether an activation/expansion step **1001** or **2001** is carried out, and how sample partitioning is carried out, one or some individual containers will have zero ASCs or zero ASCs or ASC precursor cells present, or an individual ASC or an individual ASC precursor cell present.

In one embodiment, individual single cells (e.g., individual ASCs or ASC precursors) from an expanded cell population are divided into discrete containers. In another embodiment, an average of a single ASC or ASC precursor is present in each of the containers.

Optionally, once the obtained sample is distributed into discrete containers to provide isolated subsamples, one or more of the isolated subsamples is subjected to conditions suitable for expansion of one or more ASCs or ASC precursor cells in the sample (Figure 1, **1003;** Figure 2, **2003).** For example, the EL4.B5 culture system for activation and expansion of B-cells can be employed, as known to those of ordinary skill in the art. Expansion **1003** or **2003** can be carried out with or without a feeder cell population. Examples of expansion protocols are described above for step **1001** and **2001,** and are also amenable for use at step **1003** and/or **2003.**

The cell culture systems of U.S. Patent Application Publication No. 2012/0009671 and/or similar cell culture systems may be employed.

In some embodiments, at step **1003** and/or **2003,** the exposure of cells in subsamples to cell culture conditions results in an increase in the expression levels of mRNA, and the activation of memory B-cells to differentiate them into ASCs. A benefit of an expansion step **1003** or **2003** following sample partitioning is that the cells of the subsamples are caused to divide and make multiple copies within the well or container. This results in a greater number of total antibodies secreted by the expanded populations of ASC, and maximizes the identification of unique epitopes within a screen cell population. A cell expansion step subsequent to sample partitioning may be used to generate variability in the number of copies of antibodies derived from any clone in the starting sample. This may be done deliberately by selecting expansion conditions that favor some clones or some containers. Nevertheless, even a polyclonal activation will result in some variability of expansion of each of the clones within the sample.

A functionalized substrate is used to capture secreted antibodies in one or more of the containers (Figure 1, **1004;** Figure 2, **2005).** In one embodiment, the functionalized substrate is a functionalized container surface that has been conjugated with antibody-capture proteins such as protein A, protein G, or polyclonal antibodies against immunoglobulins of one or more isotype from the species being studied.

In another embodiment, antibodies secreted by ASCs in a subsample (expanded or non-expanded subsample) are captured on a functionalized microscale bead substrate, present in the containers (Figure 1, **1004;** Figure 2, **2005).** Specifically, a functionalized substrate such as microscale beads that have been conjugated with antibody-capture proteins such as protein A, protein G, or polyclonal antibodies against human immunoglobulins, in one embodiment, are present in the containers or a subset thereof. ASCs are maintained in spatial proximity to the functionalized beads, and the beads capture the antibodies secreted by the ASCs. The microscale beads can be introduced into the discrete containers prior to the sample introduction, subsequent to the sample introduction, or simultaneously with the sample introduction (sample partitioning into subsamples). The cells in the subsample can be expanded at step **1003** or **2003** and when this step is carried out, in one embodiment, the microscale beads are introduced into the discrete containers after the subsamples are expanded. Following the capture of secreted antibodies in the chambers the antibodies may be interacted with a display library to allow for binding of the antibodies with recognized antigens in the display library. It will be known to those with ordinary skill in the art that efficient interaction of the display library with the antibodies requires that each antibody have the opportunity to interact with one or more members of the display library, and that this is determined by the volume and diversity of the display library, as well as the duration and mode of interaction. For instance, if the display library is comprised of a phage display library, the volume of the library that interacts with the captured antibody should be sufficient such that all members of the library are represented at least once. A phage display library may have a concentration of up to 10¹⁴ particles per mL (Nemudraya et al. (2016). Acta Naturae 8(1), (28)), and may easily have concentration of 10¹² particles per mL. If an interrogated ASC is analyzed in a container having a volume of 1 nL, this volume may include between approximately 10⁶ to 10⁸ phage particles. Flowing additional volume of display library through the container (e.g., microfluidic chamber) to interact with bound antibody may be performed to increase this diversity by 100 fold or more, thereby allowing for interrogation of libraries between 10⁸ and 10¹⁰. It will be appreciated by those with ordinary skill in the art that larger libraries may be interrogated through the use of larger chamber volumes or by repeated steps of incubation and flowing of additional library volume. Alternative library formats such as ribosome display libraries may be produced at higher concentrations so that library diversity is increased. Alternatively, libraries of lower total diversity but with greater concentration of each antigen may be preferred in cases where detection of binding is desired, e.g. using a fluorescently labeled reagent.

The functionalized substrate such as functionalized beads can be introduced into the containers prior to sample introduction, subsequent to sample introduction, or simultaneously with the sample. Functionalized antibody capture beads are available commercially. For example, protein A and protein G beads are available from Promega, and are amenable for use with the methods provided herein (Promega product nos. G7471, G7472, G7473, G8781, G8782 and G8783). Alternatively, container surfaces can be functionalized with an antibody capture protein prior to introducing the sample into the containers. Notably, the invention is not limited by a particular type of antibody capture bead or functionalized surface.

Secreted antibodies are captured on the functionalized surface, e.g., on the surface of a bead(s) or container surface. Moreover, as a result of distributing the sample into microscale containers, a large number of ASCs are maintained in spatial proximity to the functionalized substrate(s) that has captured the secreted antibodies. This is the case whether ASCs are present in the sample at step **1002** or **2002** or the sample is expanded at **1003** or **2003.**

Captured antibodies in one or more of the containers are exposed to antigen display library particles for a time sufficient to allow for interaction of the captured antibodies with particles of the display library (Figure 1, **1005;** Figure 2, **2006).**

The captured ASCs are exposed to a display library (Figure 1, **1005;** Figure 2, **2006)** that presents a collection of antigens to the antibodies that are captured on the solid surfaces. As discussed in further detail below, in one aspect of the invention, prior to library interrogation, captured ASCs are subjected to a functional assay to determine the presence of functional antibodies in one or more containers (e.g., Figure 2, **2005).** The results of the functional assay(s) determine which ASCs (and which containers) are interrogated with the antigen display library.

The displayed antigens in a particular library is not limited by size. For example, the displayed peptide in one embodiment is from about 10 amino acids to about 50 amino acids in length, from about 15 amino acids to about 50 amino acids in length, at least about 10 amino acids in length, etc. Size of antigens can be adjusted depending on the application studied and the method of display. Moreover, the size of a library can be increased by scrambling libraries and performing alanine scanning mutagenesis on members of the library. Displayed peptides can also be designed to overlap in sequence with other antigens.

The methods provided herein are not limited to a particular type of display library, or the number of antigens in a display library. For example, the display system can be a cell-free system, which includes libraries of (poly)peptide-nucleic acid complexes, in which each polypeptide is physically bound to its nucleic acid coding sequence, or a DNA or RNA sequence that can be used to determine its identity (sometimes referred to as a "barcode" sequence).

A barcode sequence in one embodiment, is used in the display library employed herein. In a further embodiment, the barcode sequence acts as the library source from which the displayed peptide is translated. Alternatively or additionally, the barcode sequence is used as an identifier, as described above. In another embodiment the barcode is a DNA sequence that encodes a peptide or polypeptide sequence, that may or may not include silent or non-silent mutations. Alternatively, the barcode is composed of a series of nucleotides linked to the 3' or 5' end of the nucleic acid molecules, optionally connected by a linker sequence. One of ordinary skill in the art will appreciate that when a barcode sequence is employed, it does not interfere with the expression of displayed peptides. In another embodiment, the barcode comprises a chemical or enzymatic modification, for example, a barcode comprised of a DNA sequence can be chemically linked or conjugated directly to a target protein or peptide. One method of synthesizing peptide libraries containing peptide-cDNA fusions by in vitro transcription/translation from pools of DNA templates generated by microarray-based synthesis is provided in Kozlov et al (2012) PLOS One. DOI: 10.1371/journal.pone.0037441.

Display libraries can be designed to allow for epitope mapping, i.e., linear peptide libraries can be employed to identify critical residues involved in biomolecular interactions. Alternatively or additionally, different spatially located epitopes of an antigen can be detected by using different barcodes corresponding to different spatially located epitopes of an antigen.

In yet another embodiment, a barcoded library is designed with three levels of information: (1) amino acid sequence of displayed peptide; (2) nucleic acid barcode for identification using next generation sequencing of large pools and (3) a tag/residue such as biotin for example and subsequent fluorescent detection in well of all binders using fluorescently labelled streptavidin or any other tag.

Alternatively or additionally, the library can be generated from a cell-based system, which is based on natural or synthetic nucleotide sequences which are translated and assembled in host cells (e.g., bacteriophage or phage display). Phage display libraries are generated by basic molecular biology techniques and allow for libraries having repertories on the order of 10¹⁰-10¹³ unique particles. Alternatively, more focused libraries of between 10³-10⁶ members may also be employed, as well as libraries in an intermediate range of diversity between 10⁶-10¹⁰ members.

Phage display technology is based on the ability to express foreign (poly)peptides as fusions to capsid proteins on the surface of bacteriophage. Surface display is achieved by inserting a peptide-encoding gene into the gene for a capsid structural protein. Billions of pooled peptides presented on phage particles form a phage-displayed peptide library, and in contrast to regular synthetic small molecule libraries, as many as 10¹⁰ different peptides can be screened simultaneously for the desired activity (Molek et al. (2011). Molecules 16, pp. 857-887). Lytic or filamentous phage or phagemid vectors are amenable for use herein. In one embodiment, the phage display system is based on filamentous phages in which peptides are fused to major (p8) or minor (p3) coat proteins.

Methods for producing phage display libraries are known to those of ordinary skill in the art. For example, the phage display technology originally pioneered by Cambridge Antibody Technology, as well as subsequent improvements and modification of this library, is amenable for use with the present invention. For example, see the methods described in U.S. Patent No. 5,959,108.

Library formats for use with the methods provided herein include cell display, phage display, yeast display, ribosome display, mRNA display, cDNA display or a combination thereof. Construction of these libraries is well known to those of ordinary skill in the art, for example, see Carter (2006); Nature Reviews Immunology 6(5), pp. 343-357; Hoogenboom (2005) Nature Biotechnology 23(9), pp. 1105-1116; Kowalczykowski (1994). Microbiological Reviews 58(3), pp. 401-465; Ruoslahti (1996). Annual Review of Cell and Developmental Biology 12, pp. 697-715; Sarikaya (2003). Nature Materials 2(9), pp. 577-585; Smith (1997) Chemical Reviews 97(2), pp. 391-410; Smith (1993). Methods in Enzymology 217, pp. 228-257; Willats (2001). Plant Molecular Biology 47(1-2), pp. 9-27; Winter (1994). Annual Review of Immunology 12, pp. 433-455.

In one embodiment, the display library is a magnetic particle bead display library, e.g., a nanoparticle bead display library. In this embodiment, displayed peptides and their corresponding barcodes are conjugated to a solid support such as a nanoparticle (e.g. see Huang et al. 2013, ORBIT bead display). The barcoded particles may be small enough that they remain in suspension in solution and can be easily washed away. The beads are magnetic, to allow for removal of nanoparticles with an external magnet. The strength of the magnet may be adjusted to select for affinity of the peptide interactions.

The antigen display library, in one embodiment, is a cell-free display library, e.g., a ribosome display library or other nucleic acid (cDNA or mRNA) display library.

The antigen display library in one embodiment is designed to have a known diversity of possible antigens which may be hundreds, thousands, millions, or billions of different library members. However, as a practical constraint, every library member must be represented at sufficient concentration to allow for efficient interaction of the library with the captured antibody species in a particular container.

In one embodiment, the library is a display library that presents antigens such as peptides, protein fragments or proteins that are present on the surface of cells, wherein the antigens are present in a tissue that is affected by a disorder. The antigen display library in one embodiment is designed with knowledge of epitopes that have been implicated in the disease or indication that is being studied. For example, although specific autoantigen epitopes are unknown for a number of autoimmune diseases and cancer, the study of these diseases has uncovered surface proteins that are expressed on affected cells, for example, tumor cells in the case of a particular cancer or beta cells in the case of type 1 diabetes mellitus. The antigen display library can be designed based on the knowledge of these surface proteins.

However, in another embodiment, the display library is a random display library.

The display library in one embodiment can be constructed via entire cDNA libraries from a subset of cells of interest. For example, cDNA libraries can be used from a tumor sample where autoantibodies implicated in a particular cancer are studied.

In one embodiment, the display library is pre-enriched prior to introducing it into one or more containers. For example, in one embodiment, the library is exposed to polyclonal antibodies obtained from a patient's serum, and which are immobilized on one or more solid phases, to enrich for a subset of library particles that bind to antibodies in the serum. It is this subset of particles that is distributed into the containers.

In another embodiment, proteins and peptides for use in an antigen library can be identified based on selective expression data of an autoimmune disease positive sample as compared to a negative control sample. For example, in the case of type 1 diabetes mellitus, protein (e.g., via mass spectrometry analysis) or cDNA expression data can be compared from a beta cell sample to a negative control beta cell sample from a healthy individual. The selective expression data from the positive sample can be used to generate the antigen library. Similar strategies can be used for other autoimmune diseases, or any disease where autoantigens are implicated, e.g., a particular cancer. In a further embodiment, the disorder is an autoimmune disorder. In even a further embodiment, the autoimmune disorder is an autoimmune disorder provided in Table 1. For example, in the case of type 1 diabetes mellitus, in one embodiment, the library is a phage display library that encodes proteins expressed on the surface of islet cells.

The display library particles are exposed to the captured antibodies for a sufficient time for the antibodies to capture library particles **1005** or **2006.** Following sufficient incubation to allow an interaction between the displayed antigens and the captured antibodies, and subsequent capture of the display particles, unbound library particles are washed off of the beads **1006** or **2007.** If a captured antibody binds to a specific member of the antigen library, this member associates, and remains associated with the library particle on the solid substrate that the antibody was captured. As will be apparent to one of ordinary skill in the art, the assays provided herein can be implemented to analyze hundreds to thousands to millions of individual cells, and therefore, hundreds to thousands to millions of antibody-antigen interactions. For example, the microfluidic format, described in PCT Publication No. WO 2014/153651, can be used for the methods described herein.

The display library presents a collection of antigens to the antibodies that are captured on the functionalized substrate (e.g., beads) in the containers. If a captured antibody binds to a specific member of the library, this member becomes and remains associated with that library particle. In one embodiment, the antigen capture step is performed to simultaneously analyze thousands to millions of individual cells for antibody-antigen interaction, e.g., in the microfluidic format described in PCT Publication No. WO 2014/153651.

Following sufficient incubation to allow an interaction between the displayed antigens and the captured antibodies, non-interacting display particles from the library are washed off the bound library particles (Figure 1, **1006;** Figure 2, **2007).**

The nucleic acid sequence of antibodies or antigen fragment binding portion(s) thereof, and/or antigens in containers that include captured display particles is then determined **(1007** or **2008).**

In one embodiment of the step **1007** or step **2008,** the contents of each of the containers are sequenced, followed by a determination of the containers that contain both antibody and antigen, as well as the sequences of the antibody and antigen.

In another embodiment, in order to determine which containers to sequence, containers are first visualized via a detection reagent to determine whether the respective container includes a bound display particle. The detection reagent can be an additional reagent, e.g., a fluorescently labeled secondary antibody that binds to a display particle. Alternatively, the display particle serves as the detection reagent. For example, the display particle in one embodiment is a fluorescently labeled display particle. ASCs, antibodies, display particles or a combination thereof are removed from container(s) that contain captured display particles, and subjected to sequencing reactions. The results of the sequencing reactions determine the identity of the antibody and/or its respective antigen binding partner **1007** or **2008.**

In one embodiment, following incubation of library particles in one or more containers, and one or more wash steps, a detection reagent is introduced into the one or more containers. The detection reagent allows for the identification of containers having ASCs that produce an antibody recognized a member of the antigen display library. Sequencing can then be employed to determine the antibody and/or antigen sequence(s) in the identified containers **1007** or **2008.** The detection reagent in one embodiment includes a fluorescently labeled secondary antibody that binds to a protein on the display particle. For example, in one embodiment, where the library is a phage library, the secondary antibody is a fluorescently labeled anti-phage antibody.

As provided above, however, in another embodiment, the library particles serve as the detection reagent. For example, the library particles in one embodiment are fluorescently labeled.

The containers, in one embodiment, are imaged, e.g., via fluorescence microscopy or a fluorescent plate reader, in order to detect the detection reagent and by extension, containers having an ASC that make an antibody which reacts to a member of the library.

The presence of a detection signal from the detection reagent is an indication that one or more display particles from the library includes an antigen that binds to an antibody in the particular container. As noted above, in some embodiments, more than one species of antigen display particle associates with a given antibody in a container, either because the antibody is poly-reactive to multiple species or because of non-specific interactions with the display particles and/or cells. Multiple species of antigen display particle can also be detected in one container in embodiments where different antibodies are secreted in the same container. The degree of enrichment of display particles in containers allows for identification of binding interactions that are significant. This may be facilitated by also analyzing antibody and display sequences obtained from "negative" wells in order to ascertain the background.

In another embodiment, a container is visualized via a detection reagent to determine whether it includes an antibody (or antigen fragment binding portion thereof) bound display particle(s). In a further embodiment, the detection reagent is visualized via imaging, e.g., via fluorescence microscopy or a fluorescent plate reader, to determine the identities of containers that include one or more ASCs that react with one or more members of the antigen display library. The detection reagent can be an additional reagent, e.g., a fluorescently labeled secondary antibody that binds to a display particle. Alternatively, the display particle serves as the detection reagent. For example, the display particle in one embodiment is a fluorescently labeled display particle. In a further embodiment, the displayed antigen is fluorescently labeled. Alternatively, the detection reagent comprises an enzyme chosen to act on a substrate in order to obtain an amplification of the detection signal.

In one embodiment of step **1007** or **2008,** ASCs and the antibody capture beads associated with display particles are recovered from containers and subjected to molecular biology protocol(s) to isolate and/or amplify nucleic acid for a subsequent sequencing reaction. This nucleic acid (e.g., RNA or DNA) material is sequenced using high-throughput sequencing instrumentation, to identify the sequence of the antibody(ies) and the identity of the associated antigen(s).

ASCs and/or antibodies and/or library particle(s) from containers are subjected to molecular biology protocol(s) to isolate and/or amplify nucleic acid that can be sequenced at step **1007** or **2008.** Sequencing reactions can be employed on nucleic acid in all containers, or for nucleic acid in containers that have been identified to contain antibody-display particle complexes. Sequencing of antigen and/or antibody sequences determines the identity of the relevant antigen(s) and/or antibody(s) at step **1007** or **2008.**

Types of sequencers and sequencing technologies amenable for use with the methods presented herein include, but are not limited to, the Genome Sequencer 20/FLX (commercialized by 454/Roche); MiSEQ instrument (Illumina), 'Solexa 1G' (later named 'Genome Analyzer' and commercialized by Illumina/Solexa), SOLiD^{™} system (commercialized by Applied Biosystems), and Polonator G.007 (commercialized by Dover Systems). Other protocols amenable for use with the methods provided herein include Polony sequencing, Helioscope^{™} single molecule sequencing, Lynx Therapeutics' Massively Parallel Signature Sequencing (MPSS), 454 pyrosequencing, Ion Torrent^{™} (Life Technologies), DNA nanoball sequencing (via rolling circle amplification), and VisiGen Biotechnologies approach.

In one embodiment, an Illumina MiSEQ instrument is used. High throughput sequencing protocols are well known to those of ordinary skill in the art. See, e.g., Gupta (2008). Trends in Biotechnology 26, pp. 602-611; Metzker. (2010). Nature Reviews Genetics 11, pp. 31-46; Schuster (2008). Nature Methods 5, pp. 16-18; Shendure and Ji (2008). Nat. Biotechnol. 26, pp. 1135-1145.

In order to determine container that contain captured display particles, in one embodiment, antibody-antigen complexes are recovered from the containers and subjected to sequencing reactions. In a further embodiment, ASCs from the containers having antibody-antigen complexes (or from a subset of containers) are recovered from the containers.

In microfluidic embodiments, recovery, in one embodiment, comprises piercing the respective microfluidic chamber comprising subsample comprising the one or more ASCs associated with one or more display particles, with a microcapillary and aspirating the chamber's contents or a portion thereof to obtain a recovered aspirated cell population.

Various methods for the recovery of one or more cells from specific containers are amenable for use herein. For example, a micropipette can be employed. In some embodiments, the PDMS material of certain devices provided herein enables the selective recovery of cells from any chamber by piercing the upper membrane with a microcapillary. In one embodiment, the membrane reseals or substantially reseals after piercing is complete. In one embodiment, cell recovery from a chamber is carried out based in part on the methods set forth by Lecault et al. (2011). Nature Methods 8, pp. 581-586. In one embodiment, the membrane above a particular chamber is pierced with the microcapillary and cells are aspirated. Recovered cells, antibodies and antigens can then be deposited in microfuge tubes for sequencing analysis, as known to those of ordinary skill in the art.

Recovery, in one embodiment is automated and entails the use of a robotic microcapillary instrument. However, recovery can also be accomplished manually with a microcapillary. The recovery methods provided herein in one embodiment allow for the recovery from 100 containers with >95% efficiency in about 45 minutes.

A microcapillary, in one embodiment, is used to recover one or more cells from a container. The cells in the container(s) are substantially recovered by aspirating the container contents into the microcapillary, to provide a recovered aspirated cell population. The microcapillary in one embodiment, has a diameter of from about 5 µm to about 200 µm. In a further embodiment, the microcapillary has a diameter of from about 5 µm to about 200 µm, or from about 5 µm to about 150 µm, or from about 5 µm to about 100 µm, or from about 5 µm to about 75 µm, or from about 5 µm to about 50 µm, or from about 50 µm to about 200 µm, or from about 100 µm to about 200 µm, or from about 150 µm to about 200 µm.

In some embodiments, the microcapillary has a beveled tip. In some embodiments, the microcapillary has an oval, square or circular cross section. The microcapillary in some embodiments is mounted on a robotic micromanipulation system on a microscope to provide an automated recovery apparatus. In one embodiment, the microcapillary provided herein has a single barrel. However, the microcapillary in other embodiments has multiple barrels, for example a double barrel, a triple barrel, or more than three barrels. In one embodiment the microcapillary may be used to selectively recovery a subset of cells and beads from a chamber.

In one embodiment, recovery is performed by first cutting a wall of the microfluidic chamber to create an access point and then extracting cells by aspiration using a microcapillary.

Recovery of one or more ASCs from one or more microfluidic chambers, and/or recovery of antigen display particles, in one embodiment, comprises magnetic isolation/recovery. For example, in one embodiment, a microfluidic chamber is exposed to a magnetic particle (or plurality of magnetic particles) that adheres to the one or more cells or particles (e.g., antibody capture particles) within the chamber.

Sequencing of ASC genetic material and/or displayed antigens from containers can be employed to determine whether an antibody-antigen interaction has taken place at step 1007 or **2008,** and the respective sequences of the antibody and antigen. In this embodiment, if an antigen sequence is present, it can be inferred that it binds to an antibody inside the container, because unbound library particles are removed from containers at wash step **1006** or **2007.** In one embodiment, at step **1007** or **2008,** containers having antigens that interact with antibodies are first determined via a fluorescence assay. The contents of the containers that are identified as having the interaction are harvested for nucleic acid sequencing. Any sequencing protocol can be used to identify the sequence of the antibody and/or the identity of the antigen, e.g., high-throughput sequencing or single molecule sequencing. Accordingly, the invention is not limited to the type of sequencer or sequencing methodology employed. Types of sequencers and sequencing technologies are described above.

In one embodiment, an Illumina MiSEQ instrument is used. High throughput sequencing protocols are well known to those of ordinary skill in the art. For example, see the following references for protocols amenable for use with the present invention. Gupta (2008). Trends in Biotechnology 26, pp. 602-611; Metzker. (2010). Nature Reviews Genetics 11, pp. 31-46; Schuster (2008). Nature Methods 5, pp. 16-18; Shendure and Ji (2008). Nat. Biotechnol. 26, pp. 1135-1145.

In one embodiment, cells and beads from individual containers are separated prior to a sequencing step. This separation may have the advantage that display particles associated non-specifically with the cell would not contribute to background signal in identifying the antigen.

In one embodiment, more than one species of antigen display particle may be associated with any given antibody, either because the antibody is poly-reactive to multiple antigen species or because of non-specific interactions with the display particles and/or cells, or because the bead has been exposed to more than one species of secreted antibody. However, the degree of enrichment of display particles in chambers allows for identification of binding interactions that are significant. This may be facilitated by also analyzing antibody and display sequences obtained from "negative" wells in order to ascertain the background. In one embodiment, cells and beads from individual chambers are separated prior to amplification and/or a sequencing step. This separation may have the advantage that display particles associated non-specifically with the cell would not contribute to background signal in identifying the antigen; this is a major issue in strategies, as presented below, where the antigen-antibody interaction occurs on the surface of a cell. In one embodiment this separation is achieved by removing beads from the reactors that contain the ASC. In another embodiment the separation is achieved by first capturing the antibodies on a substrate that is in contact with the supernatant containing the secreted antibodies. In another embodiment the separation is achieved by removing a volume of the supernatant from each container prior to capturing the secreted antibodies onto a solid phase, such as beads of a solid substrate, and then interacting said beads or solid substrate with the display library.

As provided herein, in the methods **1000** and **2000** described herein, the sample is distributed into a plurality of discrete microfluidic chambers. Amongst all microfluidics technologies, multilayer soft lithography (MSL) is unique in its rapid and inexpensive prototyping of devices having thousands of integrated microvalves (Thorsen et el. (2002). Science 298, pp. 58-584). These valves can be used to build higher-level fluidic components including mixers, peristaltic pumps (Unger et al. (2000). Science 7, pp. 113-116) and fluidic multiplexing structures (Thorsen et el. (2002). Science 298, pp. 58-584; Hansen and Quake (2003). Curr. Opin. Struc. Biol. 13, pp. 538-544) thus enabling high levels of integration and on-chip liquid handling (Hansen et al. (2004). Proc. Natl. Acad. Sci. U.S.A. 101, pp. 14431-1436; Maerkl and Quake (2007). Science 315, pp. 233-237).

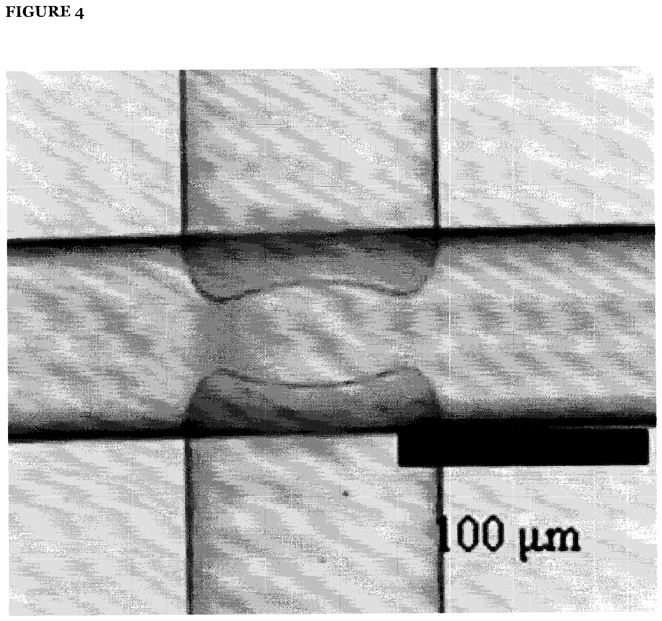

Figure 4 shows an optical micrograph of a valve made by MSL. Two crossing microfabricated channels, one "flow channel" for the active fluids (vertical) and one control channel for valve actuation (horizontal), create a valve structure. The flow channel is separated from the control channels by a thin elastomeric membrane to create a "pinch valve." Pressurization of the control channel deflects the membrane to close off the flow channel.

The chambers of a device, in one embodiment, have an average volume of from about 100 pL to about 100 nL. For example, in one embodiment, a sample is distributed into a plurality of microfluidic chambers and the average volume of the plurality of chambers is about 100 pL, about 200 pL, about 300 pL, about 400 pL, about 500 pL, about 600 pL, about 700 pL, about 800 pL, about 900 pL or about 1 nL. In another embodiment, the average volume of the microfluidic chambers is about 2 nL. In another embodiment, the average volume of the microfluidic chamber is from about 100 pL to about 100 nL, from about 100 pL to about 50 nL, from about 100 pL to about 10 nL, from about 100 pL to about 1 nL, from about 50 pL to about 100 nL, from about 50 pL to about 50 nL, from about 50 pL to about 10 nL or from about 50 pL to about 1 nL. In even another embodiment, the average volume of the microfluidic chambers is about 10 nL, about 20 nL, about 30 nL, about 40 nL, about 50 nL, about 60 nL, about 70 nL, about 80 nL, about 90 nL or about 100 nL.

The MSL fabrication process takes advantage of well-established photolithography techniques and advances in microelectronic fabrication technology. The first step in MSL is to draw a design of flow and control channels using computer drafting software, which is then printed on high-resolution masks. Silicon (Si) wafers covered in photoresist are exposed to ultraviolet light, which is filtered out in certain regions by the mask. Depending on whether the photoresist is negative or positive, either areas exposed (negative) or not (positive) crosslinks and the resist will polymerize. The unpolymerized resist is soluble in a developer solution and is subsequently washed away. By combining different photoresists and spin coating at different speeds, silicon wafers are patterned with a variety of different shapes and heights, defining various channels and chambers. The wafers are then used as molds to transfer the patterns to polydimethylsiloxane (PDMS). In one embodiment, prior to molding with PDMS and after defining photoresist layers, molds are parylene coated (chemical vapor deposited poly(p-xylylene) polymers barrier) to reduce sticking of PDMS during molding, enhance mold durability and enable replication of small features

In MSL, stacking different layers of PDMS cast from different molds on top of each other is used to create channels in overlapping "flow" and "control" layers. The two (or more) layers are bound together by mixing a potting prepolymer component and a hardener component at complementary stoichiometric ratios to achieve vulcanization. In order to create a simple microfluidic chip, a "thick" layer (e.g., between from about 200-2000 µms) is cast from the mold containing the flow layer, and the "thin" layer (e.g., between from about 25 to about 300 µms) is cast from the mold containing the control layer. After partial vulcanization of both layers, the flow layer is peeled off its mold, and aligned to the control layer (while still present on its mold, by visual inspection. The control and flow layers are allowed to bond, for example at 80 °C for about 15-60 minutes. The double slab is then peeled from the control mold, and inlet and outlet holes are punched and the double slab is bonded to a blank layer of PDMS *(i.e.,* a flat layer of PDMS with no structural features). After allowing more time to bond, the completed device is mounted on a glass slide. Fluid flow in the device is controlled using off-chip computer programmable solenoids which actuate the pressure applied to fluid in the channels of the control layer. When pressure is applied to these control channels, the flexible membrane between the overlapping orthogonal control and flow lines deflects into the flow channel, effectively valving the flow. Different combinations of these valves can be used to create peristaltic pumps, multiplexer controls and isolate different regions of the chip.

With respect to the flow layer, assay chambers and channels for controlling fluidic flow to and from the assay chambers are defined by the photoresist layers. As will be appreciated by one of skill in the art, the thickness of a photoresist layer can be controlled in part by the speed of spin coating and the particular photoresist selected for use. The bulk of the assay chambers, in one embodiment, are defined by an SU-8 100 feature which sits directly on the Si wafer. As known to those of skill in the art, SU-8 is a commonly used epoxy-based negative photoresist. Alternatively, other photoresists known to those of skill in the art can be used to define assay chambers with the heights described above. In some embodiments, the assay chambers have a height and width of 50-500 µM and 50-500 µM, respectively, as defined by the SU-8 features.

MSL fabrication techniques allow for a wide range of device densities, and chamber volumes to be fabricated. For the devices provided herein, in one embodiment, from about 2000 to about 10,000 discrete chambers are provided in a single integrated device. In one embodiment the discrete chambers are connected in a serial format. Alternatively or additionally, the discrete chambers are individually addressable. For example, a device with 4032 individual analysis chambers (average volume of 2.25 nL) connected in serial format achieve a screening throughput of approximately 100,000 cells per run, as described in PCT Publication No. WO 2014/153651, which published October 2, 2014. The integrated microfluidic valves harnessed in the devices provided herein allow for chamber isolation, and programmable washing with reagents selected from a plurality of inlets, for example from 2 to about 32 inlets, 2 to about 20 inlets, 2 to about 15 inlets, 2 to about 10 inlets, or from 2 to about 9 inlets, or from 2 to about 8 inlets, or from 2 to about 7 inlets or from 2 to about 6 inlets. Additional inlets can be provided to control valve pressure.

In one embodiment, the containers (microfluidic chambers) provided herein allow for the culture and maintenance of cells, e.g., to form an expanded lymphocyte population comprising ASCs (e.g., Figure 1, 1003; Figure 2, 2003). In one embodiment, microfluidic arrays of chambers are fabricated within a thick membrane (e.g., from about 150 µm to about 500 µm thick, about 150 µm to about 200 µm thick, about 150 µm to about 300 µm thick, about 150 µm to about 400 µm thick or about 500 µm thick) of PDMS elastomer that is overlaid a reservoir of medium, for example 1 mL of medium as described previously (Lecault et al. (2011). Nature Methods 8, pp. 581-586). The proximity of the medium reservoir (osmotic bath) to the cell chambers effectively blocks evaporation (through the gas-permeable PDMS material) and ensures robust cell viability and where cells are not fully differentiated, growth over several days, and is critical for achieving long-term culture in nL volumes with growth rates and cellular responses that are identical to microliter volume formats.

In embodiments of method **2000,** ASC subsamples are pre-enriched, prior to interrogation with a display library by functional characterization of the ASC or antibody secreted by the ASC in a particular subsample. In one embodiment of method **2000,** prior to interrogation of antibody binding to a displayed antigen, one or more functional assays are carried out on the ASC or secreted antibody to identify functional antibodies (Figure 2, **2004).** In one embodiment of the invention, after distributing the sample or expanded sample into a plurality of containers **2002,** a functional analysis is carried out to identify one or more functional ASCs that exhibits a desired property (affinity for a particular antigen, specificity for a particular antigen, etc.) (Figure 2, **2004).** In one embodiment of step **2004,** a subsample is coincubated with a target cell or a readout cell (e.g. cancer cell, bacteria cell), and detected with a secondary antibody to confirm the presence of a cell binding antibody or other functional feature. In another embodiment of step **2004,** ASCs may also be fixed and/or permeabilized to look for binding to intracellular antigens. Other functional assays include, but are not limited to a blocking assay to identify blocking peptides, protein-cell binding assays, affinity and/or specificity assays.

Additional functional assays as described herein, and also in WO 2014/153651, may be implemented at step **2004,** to detect blocking, agonist, antagonist or neutralization activity of the ASC or secreted antibody. From the functional assay, functional subsamples are identified, antibodies from the subsamples captured **2005** and subjected to further interrogation with a display library **2006.**

The functional assay can be carried out in the same container(s) as the container(s) for assessing binding to a display particle. In other words, step **2004** can be carried out in the same or different container than step **2005** and/or step **2006.** (Figure 2).

The methods provided herein allow for the initial identification of a functional ASC in the presence of other types of cells, including other lymphocytes and non-functional ASCs. The functional ASC can be present in a homogenous population of cells, a heterogeneous population or as a single cell. The functional ASC's secreted antibodies are captured for interrogation with the display library (Figure 2, **2005** and **2006).** The functional ASC(s), once identified, can be transferred to a separate container for antibody capture and subsequent library interrogation, or can be interrogated in the same container as the functional assay.

The functional assay used in the methods described herein may be varied considerably, according to the desired property the user of the method wishes to identify. As provided herein, defined medium conditions may be used to affect a polyclonal expansion and/or differentiation of ASC precursors into ASCs (Figure 2, **2001** and **2003).** In such cases possible antibody and ASC assays include without limitation, ELISPOT, fluorescent binding assays, cell binding assays, neutralization assays, surface plasmon resonance, complement fixation assays, cell-mediated cytotoxicity assays, cell binding assays, competition assays, agglutination assays, receptor agonist assays, receptor antagonist assays, antibody internalization assays, etc. In one embodiment, a functional assay is performed directly on ASCs using methods such as FACS, microscopy, colony assays, and other assays known to those of ordinary skill in the art.

In some instances, the expansion of ASCs and/or ASC precursors facilitates the analysis of functional properties within conventional cell culture formats having volumes between ~10 microliters and 10 mL. Formats may also include miniaturized cell analysis reactors including microfluidic devices, microdroplets, open microwells, plates, or semi-solid medium. Volumes of miniaturized cell analysis reactors may include approximately 100 pL, 1 nL, 10 nL, 100 nL, or 1 µL.

In one embodiment, the functional assay (including binding property screening, e.g., affinity, specificity) is performed in a microfluidic format as described in PCT Publication No. WO 2014/153651, which published October 2, 2014. The functional assays described in WO 2014/153651 can be carried out in any of the containers described herein.

In one embodiment, prior to analysis of binding to the display library, the subsample (expanded or non-expanded) or subportion thereof is subjected to a functional assay to assess the binding to one or more cell surface receptors of the cell population. Containers that include cells which bind the one or more cell surface receptors are identified and cells from those containers are processed further.

In one embodiment, the functional assay is one or more functional assays described in PCT Publication No. WO 2014/153651. In another embodiment, the functional assay is a neutralization assay, a serum bactericidal antibody assay (SBA) or an opsonophagocytic assay (OPA). For example, one or more of the functional assays described in Feavers and Walker (2010). Methods Mol. Biol. 626, pp. 199-211, can be used with the methods described herein.

In another embodiment, the functional assay is a complement dependent cytotoxicity assay (CDC) assay. In another embodiment, the functional assay is a complement-dependent cytotoxicity (CDC) assay. In one CDC embodiment, a method is provided for identifying the presence of a lymphocyte or progenitor thereof (or secreted immune receptor) that binds to a readout cell in the presence of soluble factors necessary and/or sufficient to induce lysis of a readout cell via the classic complement pathway. The assay is to determine whether an antibody secreted by a lymphocyte progenitor stimulates lysis of one or more target cells by the classic complement pathway. Cell lysis by the complement pathway is quantified according to methods known to those of skill in the art. For example, cell lysis is quantified by a clonogenic assay, by the addition of a membrane integrity dye, by the loss of intracellular fluorescent molecules or by the release of intracellular molecules in solution. The released biomolecules are measured directly in solution or captured onto readout particles in the assay container.

In another embodiment, the functional assay (Figure 2, **2004)** is an antibody-dependent cell mediated cytotoxicity (ADCC) assay. ADCC is a mechanism of cell-mediated immune defense whereby an effector cell of the immune system lyses a target cell, whose membrane-surface antigens have been bound by specific antibodies. Classical ADCC is mediated by natural killer (NK) cells. However, macrophages, neutrophils and eosinophils can also mediate ADCC, and can be provided herein as cells to be used in an ADCC functional assay. ADCC assays are known to those of ordinary skill in the art and components are commercially available. For example, the Guava Cell Toxicity Kit for Flow Cytometry (Millipore), the ADCC Reporter Bioassay Core Kit (Promega), the ADCC Assay (GenScript), the LIVE/DEAD Cell Mediated Cytotoxicity Kit (Life Technologies) and the DELFIA cell toxicity assays can be utilized in the devices provided herein.

A cell growth modulation assay can be performed as a functional assay. The cell growth modulation assay can also be performed with a single readout cell or readout particles or a heterogeneous readout cell population in a single chamber, *i.e.,* a readout cell to determine whether cell growth is modulated. As used herein, a "readout" cell or particle is any particle, including a bead or cell, e.g., a functionalized bead or a cell that reports a functionality or property, or is used in an assay to determine an ASC or antibody effect. A "readout particle" can be present as a single readout particle or within a homogeneous or heterogeneous population of readout particles within a single container (e.g., microfluidic chamber). In one embodiment, a readout particle is a bead functionalized to bind one or more antibodies secreted by an ASC, or released by an ASC upon lysis of the ASC. A single readout particle may be functionalized to capture one or more different types of biomolecules, for instance a protein and/or nucleic acid, or one or more different monoclonal antibodies.

The cell growth modulation assay, in one embodiment, is adapted to screen for cells producing biomolecules that inhibit cell growth. In another embodiment, the method is adapted to screen for cells producing molecules that modulate, *i.e.,* increase or decrease, proliferation rates of readout cells. Growth rate, in one embodiment, is measured by manual or automated cell count from light microscopy images, total fluorescence intensity of cell expressing fluorescence, average fluorescence intensity of cells labeled with a dilutive dye *(e.g.,* CFSE), nuclei staining or some other method known to those of skill in the art. Commercially available assay to measure proliferation include the alamarBlue^{®} Cell Viability Assay, the CellTrace^{™} CFSE Cell Proliferation Kit and the CellTrace^{™} Violet Cell Proliferation Kit (Life Technologies), each of which can be used with the methods described herein.

In another embodiment, an apoptosis functional assay is carried out to determine a functional subsample of ASCs. In one embodiment, the method is used to identify the presence of an antibody that induces apoptosis of a cell.

In one embodiment, an autophagy assay is carried out as the functional assay (Figure 2, **2004).** In one embodiment, microscopic imaging of the subpopulation(s) is carried out after the assay, to assess autophagy using cell lines engineered with autophagy reporters that are known to those of ordinary skill in the art (e.g., FlowCellect^{™} GFP-LC3 Reporter Autophagy Assay Kit (U20S) (EMD Millipore), Premo^{™} Autophagy Tandem Sensor RFP-GFP-LC3B Kit (Life Technologies)).

In one embodiment, a cytokine assay is performed as a functional assay on one or more subpopulations (or subsets thereof). Examples of commercially available cytokine-dependent or cytokine-sensitive cell lines for such assays include, but are not limited to TF- 1 , NR6R-3T3, CTLL-2, L929 cells, A549, HUVEC (Human Umbilical Vein Endothelial Cells), BaF3, BW5147.G.1.4.0UAR.1, (all available from ATCC), PathHunter^{®} CHO cells (DiscoveRx) and TANGO cells (Life Technologies). A person skilled in the art will understand that primary cells (e.g., lymphocytes, monocytes) may also be used as readout cells for a cytokine assay.

In one embodiment, a signaling assay is used to identify a functional cell. Activation of a signaling pathway can be visualized by expression of a fluorescent reporter, translocation of a fluorescent reporter within a cell, a change in growth rate, cell death, a change in morphology, differentiation, a change in the proteins expressed on the surface of the readout cell, etc. In one embodiment the signaling assay includes a measurement of RNA transcription from readout cells. Several engineered reporter cell lines are commercially available and can be used to implement such an assay. Examples include PathHunter cells^{®} (DiscoverRx), TANGO^{™} cells (Life Technologies) and EGFP reporter cells (ThermoScientific).

In one embodiment, the functional assay measures binding of an antibody to a cell surface protein or membrane bound or integral membrane receptor, such as a G-protein coupled receptor or other membrane bound or integral membrane protein. In another embodiment, the functional assay measures the activation of a cell signaling protein or the phosphorylation of a target protein.

The functional assay can be carried out in the same container or a different container than the container used to interrogate antibody binding to a display library. In one embodiment, the functional assay is carried out in a microfluidic format and the functional ASC(s) is recovered for further analysis, for example, for exposure to an antigen library (Figure 2, 2005, 2006). Recovery, from microfluidic devices is discussed in detail above. In another embodiment, the functional assay is carried out in a non-microfluidic format and the functional ASCs are partitioned into different containers upon completion of the functional assay. Once functional ASCs are identified (Figure 2, 2005), these cells are subjected to library interrogation as described in detail herein. The containers used to identify the functional lymphocytes (e.g., ASCs) can be further employed to interrogate these functional cells with the antigen display library. Alternatively, the functional ASCs can be further partitioned into different containers.

It is further noted that it is not necessary that every instance of a member of one library interact with every member of another library because in either library, there may be multiple instances of each library member, as well as multiple useful interactions that may be recovered without the need to have explored all possible interactions. Further, it will be appreciated that the recurrent detection of a specific antigen antibody interaction in multiple containers, even in the presence of additional antibodies and antigens, may be used to identify the specific antigen and antibody that are responsible for the detected interactions.

The embodiments illustrated and discussed in this specification are intended only to teach those skilled in the art the best way known to the inventors to make and use the invention. Modifications and variation of the above-described embodiments of the invention are possible without departing from the invention, as appreciated by those skilled in the art in light of the above teachings. It is therefore understood that, within the scope of the claims, the invention may be practiced otherwise than as specifically described.

## Claims

1. A method for determining a nucleic acid sequence encoding an antibody that interacts with one or more display particles in a sample comprising one or more antibody secreting cells (ASCs) or ASC precursor cells and the nucleic acid sequence of nucleic acid(s) used to identify the antigen(s) that interact with the antibody, the method comprising
optionally, subjecting the sample comprising the one or more ASCs or ASC precursor cells to conditions suitable for expansion of the one or more ASCs or ASC precursor cells, to form an expanded sample comprising ASCs;
distributing the sample into a plurality of microfluidic chambers to provide a plurality of subsamples, wherein each microfluidic chamber contains a single ASC or ASC precursor cell;
optionally, subjecting one or more subsamples to conditions suitable for expansion of the one or more ASCs or ASC precursor cells in the one or more subsamples to form one or more expanded subsamples comprising ASCs;
capturing antibodies, present in one or more of the subsamples, on a solid substrate in one or more of the plurality of microfluidic chambers to provide captured antibodies;
exposing the captured antibodies in one or more of the microfluidic chambers to an antigen display library comprising a plurality of display particles, for a time sufficient to allow for interaction of the captured antibodies with one or more of the plurality of display particles, wherein each display particle of the plurality of display particles comprises a (poly)peptide-nucleic acid complex, wherein the (poly)peptide is an antigen and the nucleic acid is used to determine the identity of the antigen;
capturing one or more display particles that interact with one or more of the antibodies to provide one or more captured display particles;
washing the solid substrate to remove unbound display particles;
sequencing the nucleic acid encoding the antibody that interacts with one or more of the captured display particles; and
sequencing the nucleic acid(s) used to identify the antigen(s) displayed on each on the one or more captured display particles that interacts with the antibody.

2. The method of claim 1, further comprising,
immediately prior to the capturing antibodies step, performing a functional assay on one or more of the subsamples or expanded subsamples to identify one or more functional subsamples, wherein the functional assay measures a property of an antibody secreted by an ASC in the one or more subsamples or expanded subsamples;
identifying one or more functional subsamples based on the results of the functional assay;
and wherein the capturing antibodies comprises capturing antibodies from one or more functional subsamples on a solid substrate in one or more microfluidic chambers to provide captured antibodies.

3. The method of claim 1 or 2, further comprising determining the microfluidic chambers that contain the captured display particles prior to the sequencing step.

4. The method of claim 3, wherein determining the microfluidic chambers that contain the captured display particles comprises imaging microfluidic chambers to detect a signal from a detection reagent to identify positive microfluidic chambers that include captured display particles.

5. The method of any one of claims 2-4, wherein the functional assay is an antibody affinity or specificity assay.

6. The method of any one of claims 2-5, wherein the functional assay is an antibody ELISPOT assay, a cytokine neutralization assay, a virus neutralization assay or an enzyme neutralization assay.

7. The method of any one of claims 2-6, wherein the antibodies from the one or more functional subsamples are captured in the same microfluidic chamber as the microfluidic chamber used for the functional assay.

8. The method of any one of claims 2-6, wherein the antibodies from the one or more functional subsamples are captured in a different microfluidic chamber from the microfluidic chamber used for the functional assay.

9. The method of any one of claims 1-8, comprising subjecting the sample comprising one or more antibody secreting cells (ASCs) or ASC precursor cells to conditions suitable for expansion of the one or more of the ASCs or ASC precursor cells, to form an expanded sample comprising ASCs.

10. The method of any one of claims 1-9, wherein one or more subsamples are subjected to conditions suitable for expansion of one or more antibody secreting cells (ASCs) or ASC precursor cells in the one or more subsamples to form one or more expanded subsamples comprising ASCs.

11. The method of any one of claims 1-10, wherein the conditions suitable for expansion comprise conditions suitable for polyclonal activation or conditions suitable for antigen-specific activation.

12. The method of any one of claims 1-11, wherein the conditions suitable for expansion comprise the use of an EL-4-B5 cell line as a feeder layer, the use of CD40 ligand, treatment with Epstein Barr virus, one or more toll like receptor agonists, phorbol 12-myristate 13-acetate (PMA) in combination with ionomycin or phytohaemagglutinin (PHA) activation, or the use of irradiated allogeneic peripheral blood mononuclear cells (PBMC) in combination with soluble anti-CD3 mAB.

13. The method of any one of claims 1-12, wherein the antigen display library is a phage display library, yeast display library, ribosome display library, mRNA display library, cDNA display library, or a combination thereof.

14. The method of any one of claims 1-13, wherein the nucleic acid sequence used to identify the antigen is the nucleic acid coding sequence of the antigen.

15. The method of any one of claims 1-13, wherein the nucleic acid sequence used to identify the antigen is a barcode sequence used to determine the identity of the antigen.

## Patentansprüche

1. Verfahren zum Bestimmen einer Nukleinsäuresequenz, die einen Antikörper kodiert, der mit einem oder mehreren Anzeigepartikeln in einer Probe interagiert, die eine oder mehrere Antikörper-sezernierende Zellen (ASCs) oder ASC-Vorläuferzellen umfasst, und der Nukleinsäuresequenz von Nukleinsäure(n), die zum Identifizieren des Antigens oder der Antigene verwendet werden, die mit dem Antikörper interagieren, wobei das Verfahren umfasst
optional Aussetzen der Probe, die die eine oder mehreren ASCs oder ASC-Vorläuferzellen umfasst, Bedingungen, die für die Expansion der einen oder mehreren ASCs oder ASC-Vorläuferzellen geeignet sind, um eine expandierte
Probe zu bilden, die ASCs umfasst;
Verteilen der Probe in eine Vielzahl mikrofluidischer Kammern, um eine Vielzahl von Teilproben bereitzustellen, wobei jede mikrofluidische Kammer eine einzelne ASC oder ASC-Vorläuferzelle enthält;
optional Aussetzen einer oder mehrerer Teilproben Bedingungen, die zum Expandieren der einen oder mehreren ASCs oder ASC-Vorläuferzellen in der einen oder den mehreren Teilproben geeignet sind, um eine oder mehrere expandierte Teilproben zu bilden, die ASCs umfassen;
Einfangen von Antikörpern, die in einer oder mehreren der Teilproben vorhanden sind, auf einem festen Substrat in einer oder mehreren der Vielzahl mikrofluidischer Kammern, um eingefangene Antikörper bereitzustellen;
Aussetzen der eingefangenen Antikörper in einer oder mehreren der mikrofluidischen Kammern einer Antigen-Anzeigebibliothek, die eine Vielzahl von Anzeigepartikeln umfasst, für eine Zeit, die ausreicht, um eine Wechselwirkung der eingefangenen Antikörper mit einem oder mehreren der Vielzahl von Anzeigepartikeln zu ermöglichen, wobei jedes Anzeigepartikel der Vielzahl von Anzeigepartikeln einen (Poly)peptid-Nukleinsäure-Komplex umfasst, wobei das (Poly)peptid ein Antigen ist und die Nukleinsäure zum Bestimmen der Identität des Antigens verwendet wird;
Einfangen eines oder mehrerer Anzeigepartikel, die mit einem oder mehreren der Antikörper interagieren, um ein oder mehrere eingefangene Anzeigepartikel bereitzustellen;
Waschen des festen Substrats, um ungebundene Anzeigepartikel zu entfernen;
Sequenzieren der Nukleinsäure, die den Antikörper kodiert, der mit einer oder mehreren der eingefangenen Anzeigepartikel interagiert, und
Sequenzieren der Nukleinsäure(n), die zum Identifizieren des Antigens oder der Antigene verwendet werden, die auf jedem des einen oder der mehreren eingefangenen Anzeigepartikel angezeigt werden, die mit dem Antikörper interagieren.

2. Verfahren nach Anspruch 1, ferner umfassend,
unmittelbar vor dem Schritt des Einfangens von Antikörpern, Durchführen eines funktionellen Assays an einer oder mehreren der Teilproben oder expandierten Teilproben, um eine oder mehrere funktionelle Teilproben zu identifizieren, wobei der funktionelle Assay eine Eigenschaft eines Antikörpers misst, der von einer ASC in der einen oder den mehreren Teilproben oder expandierten Teilproben sezerniert wird;
Identifizieren einer oder mehrerer funktioneller Teilproben auf Basis der Ergebnisse des funktionellen Assays;
und wobei das Einfangen von Antikörpern das Einfangen von Antikörpern aus einer oder mehreren funktionellen Teilproben auf einem festen Substrat in einer oder mehreren mikrofluidischen Kammern umfasst, um eingefangene Antikörper bereitzustellen.

3. Verfahren nach Anspruch 1 oder 2, ferner umfassend Bestimmen der mikrofluidischen Kammern, die die eingefangenen Anzeigepartikel enthalten, vor dem Schritt des Sequenzierens.

4. Verfahren nach Anspruch 3, wobei Bestimmen der mikrofluidischen Kammern, die die eingefangenen Anzeigepartikel enthalten, Abbilden mikrofluidischer Kammern zum Nachweisen eines Signals von einem Nachweisreagenz umfasst, um positive mikrofluidische Kammern zu identifizieren, die eingefangene Anzeigepartikel enthalten.

5. Verfahren nach einem der Ansprüche 2 bis 4, wobei der funktionelle Assay ein Antikörper-Affinitäts- oder -Spezifitätsassay ist.

6. Verfahren nach einem der Ansprüche 2 bis 5, wobei der funktionelle Assay ein Antikörper-ELISPOT-Assay, ein Zytokin-Neutralisierungsassay, ein Virus-Neutralisierungsassay oder ein Enzym-Neutralisierungsassay ist.

7. Verfahren nach einem der Ansprüche 2 bis 6, wobei die Antikörper aus der einen oder den mehreren funktionellen Teilproben in derselben mikrofluidischen Kammer wie der für den funktionellen Assay verwendeten mikrofluidischen Kammer eingefangen werden.

8. Verfahren nach einem der Ansprüche 2 bis 6, wobei die Antikörper aus der einen oder den mehreren funktionellen Teilproben in einer anderen mikrofluidischen Kammer als der für den funktionellen Assay verwendeten mikrofluidischen Kammer eingefangen werden.

9. Verfahren nach einem der Ansprüche 1 bis 8, umfassend Aussetzen der Probe, die eine oder mehrere Antikörper-sezernierende Zellen (ASC) oder ASC-Vorläuferzellen umfasst, Bedingungen, die für die Expansion der einen oder mehreren ASCs oder ASC-Vorläuferzellen geeignet sind, um eine expandierte Probe zu bilden, die ASCs umfasst.

10. Verfahren nach einem der Ansprüche 1 bis 9, wobei eine oder mehrere Teilproben Bedingungen ausgesetzt werden, die für die Expansion einer oder mehrerer Antikörper-sezernierender Zellen (ASCs) oder ASC-Vorläuferzellen in der einen oder den mehreren Teilproben geeignet sind, um eine oder mehrere expandierte Teilproben zu bilden, die ASCs umfassen.

11. Verfahren nach einem der Ansprüche 1 bis 10, wobei die für die Expansion geeigneten Bedingungen Bedingungen umfassen, die für eine polyklonale Aktivierung geeignet sind, oder Bedingungen, die für eine antigenspezifische Aktivierung geeignet sind.

12. Verfahren nach einem der Ansprüche 1 bis 11, wobei die für die Expansion geeigneten Bedingungen die Verwendung einer EL-4-B5-Zelllinie als Feederschicht, die Verwendung von CD40-Ligand, Behandlung mit Epstein-Barr-Virus, einem oder mehreren Toll-like-Rezeptor-Agonisten, Phorbol-12-Myristat-13-Acetat-(PMA-) in Kombination mit lonomycin- oder Phytohämagglutinin-Aktivierung (PHA-Aktivierung) oder die Verwendung von bestrahlten allogenen peripheren mononukleären Blutzellen (PBMC) in Kombination mit löslichem Anti-CD3-mAB umfassen.

13. Verfahren nach einem der Ansprüche 1 bis 12, wobei die Antigen-Anzeigebibliothek eine Phagen-Anzeigebibliothek, Hefe-Anzeigebibliothek, Ribosomen-Anzeigebibliothek, mRNA-Anzeigebibliothek, cDNA-Anzeigebibliothek oder eine Kombination davon ist.

14. Verfahren nach einem der Ansprüche 1 bis 13, wobei die zum Identifizieren des Antigens verwendete Nukleinsäuresequenz die Nukleinsäure-Kodiersequenz des Antigens ist.

15. Verfahren nach einem der Ansprüche 1 bis 13, wobei die zum Identifizieren des Antigens verwendete Nukleinsäuresequenz eine Barcode-Sequenz ist, die zum Bestimmen der Identität des Antigens verwendet wird.

## Revendications

1. Procédé de détermination d'une séquence d'acides nucléiques codant pour un anticorps qui interagit avec une ou plusieurs particules d'affichage dans un échantillon comprenant une ou plusieurs cellules sécrétrices d'anticorps (ASC) ou cellules précurseurs d'ASC et de la séquence d'acides nucléiques d'acide(s) nucléique(s) utilisé(s) pour identifier le(s) antigène(s) qui interagissent avec l'anticorps, le procédé comprenant
facultativement, la soumission de l'échantillon comprenant la ou les ASC ou cellules précurseurs d'ASC à des conditions appropriées pour l'expansion de la ou des ASC ou cellules précurseurs d'ASC, pour former un échantillon expansé comprenant des ASC ;
la distribution de l'échantillon dans une pluralité de chambres microfluidiques pour fournir une pluralité de sous-échantillons, dans lequel chaque chambre microfluidique contient une unique ASC ou cellule précurseur d'ASC ;
facultativement, la soumission d'un ou plusieurs sous-échantillons à des conditions appropriées pour l'expansion de la ou des ASC ou cellules précurseurs d'ASC dans le ou les sous-échantillons pour former un ou plusieurs sous-échantillons expansés comprenant des ASC ;
la capture d'anticorps, présents dans un ou plusieurs des sous-échantillons, sur un substrat solide dans une ou plusieurs parmi la pluralité de chambres microfluidiques pour fournir des anticorps capturés ;
l'exposition des anticorps capturés dans une ou plusieurs des chambres microfluidiques à une bibliothèque d'affichage d'antigènes comprenant une pluralité de particules d'affichage, pendant une période suffisante pour permettre une interaction des anticorps capturés avec une ou plusieurs parmi la pluralité de particules d'affichage, dans lequel chaque particule d'affichage parmi la pluralité de particules d'affichage comprend un complexe (poly)peptide-acide nucléique, dans lequel le (poly)peptide est un antigène et l'acide nucléique est utilisé pour déterminer l'identité de l'antigène ;
la capture d'une ou plusieurs particules d'affichage qui interagissent avec un ou plusieurs des anticorps pour fournir une ou plusieurs particules d'affichage capturées ;
le lavage du substrat solide pour éliminer les particules d'affichage non liées ;
le séquençage de l'acide nucléique codant pour l'anticorps qui interagit avec une ou plusieurs des particules d'affichage capturées ; et
le séquençage du ou des acide(s) nucléique(s) utilisé(s) pour identifier le ou les antigène(s) affiché(s) sur chacune parmi la ou les particules d'affichage capturées qui interagit avec l'anticorps.

2. Procédé selon la revendication 1, comprenant en outre,
immédiatement avant l'étape de capture d'anticorps, la mise en œuvre d'un dosage fonctionnel sur un ou plusieurs des sous-échantillons ou sous-échantillons expansés pour identifier un ou plusieurs sous-échantillons fonctionnels, dans lequel le dosage fonctionnel mesure une propriété d'un anticorps sécrété par une ASC dans le ou les sous-échantillons ou sous-échantillons expansés ;
l'identification d'un ou plusieurs sous-échantillons fonctionnels sur la base des résultats du dosage fonctionnel ;
et dans lequel la capture d'anticorps comprend la capture d'anticorps provenant d'un ou plusieurs sous-échantillons fonctionnels sur un substrat solide dans une ou plusieurs chambres microfluidiques pour fournir des anticorps capturés.

3. Procédé selon la revendication 1 ou 2, comprenant en outre la détermination des chambres microfluidiques qui contiennent les particules d'affichage capturées, avant l'étape de séquençage.

4. Procédé selon la revendication 3, dans lequel la détermination des chambres microfluidiques qui contiennent les particules d'affichage capturées comprend l'imagerie de chambres microfluidiques pour détecter un signal provenant d'un réactif de détection pour identifier des chambres microfluidiques positives qui incluent des particules d'affichage capturées.

5. Procédé selon l'une quelconque des revendications 2 à 4, dans lequel le dosage fonctionnel est un dosage d'affinité ou spécificité d'anticorps.

6. Procédé selon l'une quelconque des revendications 2 à 5, dans lequel le dosage fonctionnel est un dosage ELISPOT d'anticorps, un dosage de neutralisation de cytokine, un dosage de neutralisation de virus ou un dosage de neutralisation d'enzyme.

7. Procédé selon l'une quelconque des revendications 2 à 6, dans lequel les anticorps provenant du ou des sous-échantillons fonctionnels sont capturés dans la même chambre microfluidique que la chambre microfluidique utilisée pour le dosage fonctionnel.

8. Procédé selon l'une quelconque des revendications 2 à 6, dans lequel les anticorps provenant du ou des sous-échantillons fonctionnels sont capturés dans une chambre microfluidique différente de la chambre microfluidique utilisée pour le dosage fonctionnel.

9. Procédé selon l'une quelconque des revendications 1 à 8, comprenant la soumission de l'échantillon comprenant une ou plusieurs cellules sécrétrices d'anticorps (ASC) ou cellules précurseurs d'ASC à des conditions appropriées pour une expansion de celle(s) des ASC ou cellules précurseurs d'ASC, pour former un échantillon expansé comprenant des ASC.

10. Procédé selon l'une quelconque des revendications 1 à 9, dans lequel un ou plusieurs sous-échantillons sont soumis à des conditions appropriées pour une expansion d'une ou plusieurs cellules sécrétrices d'anticorps (ASC) ou cellules précurseurs d'ASC dans le ou les sous-échantillons pour former un ou plusieurs sous-échantillons expansés comprenant des ASC.

11. Procédé selon l'une quelconque des revendications 1 à 10, dans lequel les conditions appropriées pour une expansion comprennent des conditions appropriées pour une activation polyclonale ou des conditions appropriées pour une activation spécifique à l'antigène.

12. Procédé selon l'une quelconque des revendications 1 à 11, dans lequel les conditions appropriées pour une expansion comprennent l'utilisation d'une lignée cellulaire EL-4-B5 en tant que couche nourricière, l'utilisation d'un ligand CD40, un traitement avec un virus d'Epstein-Barr, un ou plusieurs agonistes de récepteur de type toll, une activation de phorbol 12-myristate 13-acétate (PMA) en combinaison avec de l'ionomycine ou de la phytohémagglutinine (PHA), ou l'utilisation de cellules mononucléaires de sang périphérique (PBMC) allogènes irradiées en combinaison avec un AcM anti-CD3 soluble.

13. Procédé selon l'une quelconque des revendications 1 à 12, dans lequel la bibliothèque d'affichage d'antigènes est une bibliothèque d'affichage de phages, une bibliothèque d'affichage de levures, une bibliothèque d'affichage de ribosomes, une bibliothèque d'affichage d'ARNm, une bibliothèque d'affichage d'ADNc, ou une combinaison de celle-ci.

14. Procédé selon l'une quelconque des revendications 1 à 13, dans lequel la séquence d'acides nucléiques utilisée pour identifier l'antigène est la séquence de codage d'acide nucléique de l'antigène.

15. Procédé selon l'une quelconque des revendications 1 à 13, dans lequel la séquence d'acides nucléiques utilisée pour identifier l'antigène est une séquence de code-barres utilisée pour déterminer l'identité de l'antigène.
